(19)

Europäisches Patentamt

European Patent Office

Office européen des brevets

(11) **EP 4 534 186 A1**

(12) **EUROPEAN PATENT APPLICATION**
published in accordance with Art. 153(4) EPC

(43) Date of publication:
**09.04.2025 Bulletin 2025/15**

(21) Application number: **22943917.9**

(22) Date of filing: **23.09.2022**

(51) International Patent Classification (IPC):
**B01D 67/00** (2006.01)   **B01D 71/06** (2006.01)
**C07K 1/34** (2006.01)   **C07K 14/765** (2006.01)

(52) Cooperative Patent Classification (CPC):
**B01D 67/00; B01D 71/06; C07K 1/34; C07K 14/765**

(86) International application number:
**PCT/KR2022/014223**

(87) International publication number:
**WO 2023/229110 (30.11.2023 Gazette 2023/48)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **27.05.2022 KR 20220065520**

(71) Applicant: **CALTH. Inc.**
**Seongnam-si, Gyeonggi-do 13449 (KR)**

(72) Inventors:
• **LEE, Jeong Hoon**
**Seongnam-si, Gyeonggi-do 13646 (KR)**
• **PARK, Seong Jun**
**Seoul 01887 (KR)**
• **LEE, Seung Min**
**Seoul 02476 (KR)**

(74) Representative: **Snaith, James Michael et al**
**Kilburn & Strode LLP**
**Lacon London**
**84 Theobalds Road**
**London WC1X 8NL (GB)**

(54) **SELECTIVE CONCENTRATION FILTER FOR SAMPLE, METHOD FOR MANUFACTURING CONCENTRATION FILTER, AND SAMPLE CONCENTRATION DEVICE COMPRISING SAME**

(57)   A concentration filter for selectively concentrating a sample, a method for manufacturing the concentration filter, and a sample concentration device are disclosed herein. The selective concentration filter of the present invention comprises: a base having pores; and a selective permeable membrane which is coated on the base and which comprises vesicles, wherein the selective permeable membrane concentrates an analyte to be analyzed from a sample and allows at least a portion of the remaining substances, other than the analyte, to be permeated through a pore structure. According to the present invention, an analyte to be analyzed can be easily concentrated through a simplified structure.

Figure 1

EP 4 534 186 A1

## Description

### Technical Field

The technical field to which the present invention belongs relates to a selective concentration device, a selective concentration filter, and a method for manufacturing a selective concentration filter. The present invention is related to research supported by the Bio and Medical Technology Development Program (No. 2021M3E5E3080743) of the National Research Foundation of Korea (NRF) supported by the Korean government (Ministry of Science and ICT).

### Background Art

The contents described in this section simply provide background information for the present embodiment and do not constitute prior art.

Modern medicine aims not simply to extend lifespan, but to realize the extension of healthy lifespan by living a long and healthy lifespan. Therefore, the paradigm of future medicine is changing from being centered on therapeutic medicine to implementing the 3P of preventive medicine, predictive medicine, and personalized medicine. In order to realize this concretely, early detection and early treatment of diseases are becoming very important means, and research on biomarkers is being conducted very actively as a means for this.

A biomarker refers to a marker that can distinguish between normal and pathological conditions, predict treatment responses, and objectively measure them. Nucleic acids (DNA, RNA), proteins, lipids, metabolites, and changes in their patterns are used as biomarkers. In other words, simple substances such as blood glucose for diagnosing diabetes, and genes such as the BCR-ABL gene fusion for chronic myeloid leukemia, which is the treatment target of Gleevec, are all biomarkers and are biomarkers that are actually used in clinical practice.

The onset and progression of a disease can be identified by analyzing nucleic acids or proteins. Technologies and devices for protein analysis have the problem that they are difficult to manufacture and are relatively expensive due to the use of nanotechnology, making them difficult to popularize. In addition, there is a disadvantage that protein analysis devices require highly sensitive sensors or that accurate analysis is difficult with a small amount of sample. A representative method for detecting nucleic acids or proteins is the lateral flow analysis method using chromatography. This lateral flow analysis method is used in various fields such as pregnancy diagnosis.

### Prior Art Document

### Patent Document

(Patent Document 1) Korean Patent Application Publication No. 10-2021-0124410 (October 14, 2021)
(Patent Document 2) Korean Patent Application Publication No. 10-2021-0129131 (October 27, 2021)
(Patent Document 3) Korean Patent Application Publication No. 10-2019-0127690 (November 13, 2019)

### Detailed Description of Invention

### Technical Problem

The embodiments of the present invention have a problem in that a filter that simply uses a pore size concentrates other unnecessary biomolecules of similar size, and in order to solve this problem, the main purpose is to provide a concentration filter that selectively removes a specific substance while concentrating the analyte through a water channel protein (aquaporin), a phospholipid layer, and a substance transport protein of a cell membrane component fused to a porous membrane.

Other unspecified purposes of the present invention can be additionally considered within the scope that can be easily inferred from the detailed description and effects thereof below.

### Solution to Problem

In orde to achieve the object of the present invention described above, the selective concentration filter for a sample comprises: a base having pores; and a selective permeable membrane which is coated on the base and which comprises vesicles, wherein the selective permeable membrane concentrates an analyte to be analyzed from a sample and allows at least a portion of the remaining substances, other than the analyte, to be permeated through a pore structure.

In the present invention, the surface of the selective permeable membrane may be hydrophilic and have a negative charge. In the present invention, the pore of the base may be selected so as to concentrate the analyte by considering at least one property selected from the group consisting of the size of the analyte, the charge of the analyte, and the affinity with the solvent. The pore diameter of the base is preferably in the range of 10 nm to 150 m.

In the present invention, the remaining substance of the sample may comprise a fixed separation substance and a dynamic separation substance, and the degree of permeation of the dynamic separation substance may depend on the spacing of the phospholipids of the cell membrane component in a first pressure section or a second pressure section distinguished according to a reference pressure applied to the selective permeable membrane.

Preferably, the remaining substance of the sample may further comprise a buffer solution, and the degree of permeation of the dynamic separation substance may depend on the hydrogen ion concentration of the buffer solution in a first hydrogen ion concentration section or a second hydrogen ion concentration section distinguished according to the reference hydrogen ion concentration.

In order to achieve another object of the present invention, a sample concentration device comprises: a selective concentration unit comprising a selective concentration filter and a concentration filter holder surrounding the selective concentration filter; and a body comprising a coupling unit that is coupled to the selective concentration filter as necessary, and having a channel formed therein for supplying a sample to be concentrated to the selective concentration filter module. Here, the selective concentration filter comprises: a base having pores; and a selective permeable membrane which is coated on the base and which comprises vesicles, wherein the selective permeable membrane does not allow an analyte to be analyzed from a sample to be permeated and allows at least a portion of the remaining substances, other than the analyte, to be permeated through the pores.

In the present invention, the concentration filter holder may further comprise a first passage and a second passage formed in a space separated from each other for the input or discharge of the sample. The first passage may be located on the selective permeable membrane of the selective concentration filter, and the second passage may be located on the base.

In the present invention, the concentration filter holder may further comprise: a first holder having the first passage; and a second holder having the second passage, and the selective concentration filter may be located between the first holder and the second holder.

In addition, the sample concentration device may further comprise a fixing unit that seals the selective concentration filter and a supporting unit that supports the selective concentration filter, which are located between the first holder and the second holder. The first passage is for injecting the sample, and the second passage is for discharging at least a portion of the remaining substances that has permeated through the pores to the outside.

Preferably, the sample concentration device may further comprise a first pressure providing unit that applies pressure to the sample to inject the sample through the first passage and provides pressure to discharge at least a portion of the remaining substances to the outside through the second passage, and the body may further comprise a first channel through which the first pressure providing unit can move.

Preferably, the sample concentration device may further comprise a second pressure providing unit that applies pressure to the selective concentration filter through the second passage to discharge the concentrated analyte to the outside through the first passage. The body may further comprise a second channel through which the second pressure providing unit can move.

Preferably, the body may further comprise a coupling unit that surrounds and supports the first pressure providing unit and the second pressure providing unit, and can be selectively coupled to the first passage or the second passage.

Preferably, the surface of the selective permeable membrane may be hydrophilic and have a negative charge, and the pore may be selected so as to concentrate the analyte by considering at least one property selected from the group consisting of the size of the analyte, the charge of the analyte, and the affinity with the solvent.

In order to achieve another object of the present invention, a method for manufacturing a selective concentration filter comprises: a step of preparing a cell membrane mixture solution comprising a cell membrane comprising a vesicle and a buffer solution; a step of applying the cell membrane mixture solution to a base having pores; and a step of discharging at least a portion of the buffer solution through the pore structure to the outside and fixing the cell membrane on the base.

The cell membrane vesicles may be obtained by ultrasonicating red blood cell membranes, and the cell membrane vesicles in the cell membrane mixture solution may be 1 % (v/v) to 4 % (v/v).

In addition, the method may further comprise, following the coating step, a step of heat treating at 40 °C to 60 °C.

In order to achieve another object of the present invention, a method for concentrating a sample using a selective concentration device for a sample that comprises a selective concentration filter; and a concentration filter holder surrounding the selective concentration filter comprises: a step of injecting the sample through the first passage and discharging at least a portion of the remaining substances that has permeated through the pore structure to the outside through the second passage; and a step of applying pressure to the selective concentration filter through the second passage to discharge at least a portion of the analyte concentrated in the selective permeable membrane through the first passage.

## Effects of Invention

As described above, according to the embodiments of the present invention, there is an effect of discharging water through a selective permeable structure in which a porous membrane and a cell membrane are laminated, and concentrating the analyte while selectively removing specific substances.

According to the embodiments of the present invention, there is an effect of dynamically controlling the permeability of a substance in a concentration filter by controlling the pressure applied to the migration path of a selective permeable structure in which a porous membrane and a cell membrane are laminated and/or the pH condition of a sample buffer.

Even if an effect is not explicitly mentioned herein, the effect described in the following specification and its temporary effect expected by the technical features of the present invention are treated as described in the specification of the present invention.

## Brief Description of Drawings

Figures 1 and 2 are diagrams illustrating a method for manufacturing a selective concentration filter according to one embodiment of the present invention.

Figures 3 and 4 are diagrams illustrating a step of preparing a cell membrane in a method for manufacturing a selective concentration filter according to one embodiment of the present invention.

Figures 5 to 9 are diagrams illustrating a surface according to the concentration of a selective concentration filter produced by a method for manufacturing a selective concentration filter according to one embodiment of the present invention.

Figures 10 to 12 are diagrams illustrating a surface potential according to the concentration of a selective concentration filter produced by a method for manufacturing a selective concentration filter according to one embodiment of the present invention.

Figure 13 is a diagram illustrating a concentration ratio according to the concentration of a selective concentration filter produced by a method for manufacturing a selective concentration filter according to one embodiment of the present invention.

Figure 14 is a reference diagram illustrating an electron microscope image of a red blood cell membrane vesicle according to one embodiment of the present invention.

Figures 15 to 19 are diagrams illustrating a sample concentration device according to another embodiment of the present invention.

Figures 20 and 21 are diagrams illustrating a selective concentration filter according to another embodiment of the present invention.

Figures 22 and 23 are diagrams illustrating a surface of a selective concentration filter according to another embodiment of the present invention.

Figures 24 to 28 are diagrams illustrating a concentration ratio of a selective concentration filter according to another embodiment of the present invention.

Figure 29 is a diagram illustrating a contact angle of a selective concentration filter according to another embodiment of the present invention.

Figures 30 to 32 are diagrams illustrating a concentration ratio of a selective concentration filter according to another embodiment of the present invention that can be adjusted using the sample properties.

Figures 33 to 38 are diagrams illustrating a concentration ratio that can be adjusted using pressure properties applied to a selective concentration filter according to another embodiment of the present invention.

Figures 39 to 41 are diagrams illustrating a concentration ratio of a selective concentration filter according to another embodiment of the present invention that can be adjusted using hydrogen ion concentration properties of a sample buffer.

Figure 42 is a flow chart illustrating the concentration characteristics and permeation characteristics of a cell membrane according to pressure, size, and charge.

Figure 43 illustrates the results obtained by comparing the sensitivity between the preprocessing system of BEETLES[2] according to one embodiment of the present invention and the existing LFA.

Figure 44 illustrates the results of the selectivity test of LFA, and Figure 45 illustrates the results of the molecular diagnosis application (RT-PCR).

Figure 46 is a configuration diagram illustrating a sample concentration device according to another embodiment of the present invention. Figure 47 is a cross-sectional diagram of a sample concentration device of Figure 46.

Figure 48 is a reference diagram for explaining the concept of concentrating a sample through a sample concentration device.

Figure 49 is a reference diagram illustrating a sample concentration and concentrated sample supply process using a sample concentration device according to one embodiment of the present invention.

Figure 50 illustrates an example of a field of use of the selective concentration filter of the present invention and a sample concentration device comprising the same.

## Mode for Carrying out the Invention

Hereinafter, in explaining the present invention, if it is judged that the related known functions are obvious to those of ordinary skill in the art and may unnecessarily obscure the gist of the present invention, the detailed description thereof will be omitted, and some embodiments of the present invention will be described in detail through exemplary drawings.

Since existing filters control the pore size to concentrate only by size, there is a problem that unnecessary biomolecules are also concentrated if the size is similar.

In order to solve this problem, in the present embodiment, concentration can be achieved by removing only water through a hydrophilic porous membrane (aquaporin that selectively allows only water in the red blood cell membrane to pass through by coating the red blood cell membrane thereon). It is possible to selectively separate and concentrate biomolecules through the aquaporin, phospholipid layer, and various transport proteins of the red blood cell membrane, and it is possible to control the concentration characteristics through the cell membrane by using pressure changes and charge changes (or pH concentration). It is possible to apply a method of controlling the charge or pH concentration under constant pressure conditions or controlling the pressure under constant charge or pH concentration. It is also possible to control the charge or pH concentration while controlling the pressure conditions.

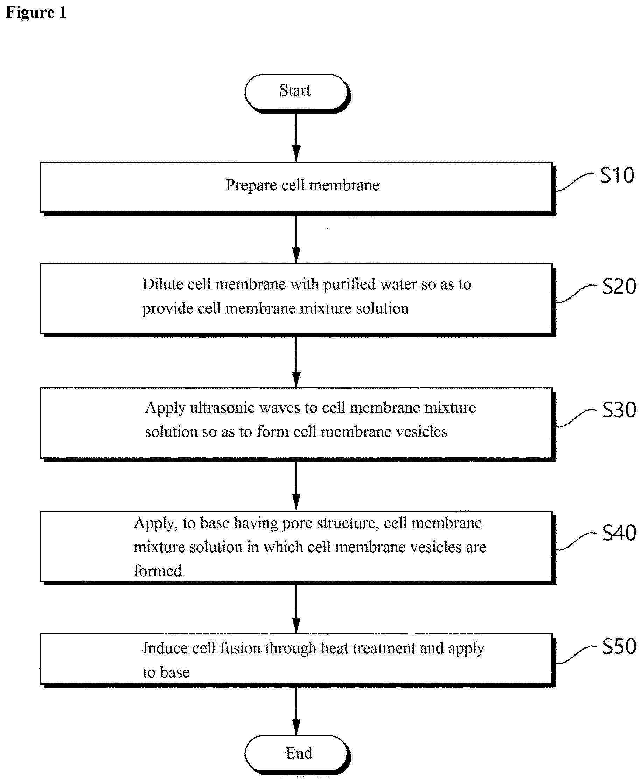

Figure 1 is a diagram illustrating one embodiment of a method for manufacturing a selective concentration filter according to one embodiment of the present invention.

Step S10 is a step of preparing a cell membrane. The cell membrane is, for example, a red blood cell membrane extracted from human or animal blood. Preferably, it is desirable to separate and remove plasma and buffy coat from the red blood cell membrane, and process it so that the removed red blood cell membrane components are included in the supernatant. The cell membrane is a membrane surrounding the cytoplasm and has a structure in which protein molecules are inserted or attached within a phospholipid bilayer. The cell membrane functions as a selective permeable membrane.

Step S20 is a step of diluting the cell membrane treated as above with purified water so as to provide a cell membrane mixture solution. Preferably, the cell membrane mixture solution comprises the cell membrane according to step S10 and a buffer solution. The buffer solution may be purified water, phosphate buffered physiological saline, etc.

Step S30 is a step of applying ultrasonic waves to the cell membrane mixture solution. As ultrasonic waves are applied to the cell membrane mixture solution, cell membrane vesicles are further formed.

Step S40 is a step of applying a cell membrane mixture solution comprising the cell membrane in which vesicles are formed through step S30 to the base. Here, the base has a pore structure and is preferably in the shape of a sheet. For example, the base may be an AAO membrane (6809-6002, Whatman, UK), but is not limited thereto. As a base, PCTE and PES membranes may also be applied. The base can be treated so that at least one surface is sufficiently covered with the cell membrane mixture solution, for example by dropping the cell membrane mixture solution on at least one surface of the base, as a method of applying the cell membrane mixture solution to the base. In addition, for uniformity of coating, Meniscuscoating, spin-coating, and the like may be applied.

Step S50 is a step of fixing the cell membrane component to at least one surface of the base through heat treatment on the base in which the cell membrane mixture solution is smeared. In this step, it is preferable to induce cell fusion through additional heat treatment. The coating in this step is a process in which the red blood cell membrane existing in the form of a vesicle is fixed to the surface of the base by changing into a multiple bilayer form. In the present embodiment, the cell membrane is a concept of concentration through a change in the distance between aquaporin, a water transport protein, and a transport protein that transports a specific protein, and phospholipids according to pressure. As the cell membrane, not only human cell membranes but also animal, plant, and bacterial cell membranes can be applied. An artificial cell membrane can be applied as the cell membrane. An artificial cell membrane is a cell membrane that is not a naturally occurring cell membrane but is formed artificially by forming components.

The cell membrane may be in the form of a sheet or may have an unspecified shape. Since the cell membrane itself has an unspecified shape, a vesicle shape is advantageous when coating the cell membrane on a porous membrane. A cell membrane vesicle is a concept of a cell membrane that is slightly deformed in shape. The cell membrane can be transformed into a vesicle shape through ultrasonic treatment. Extrusion can be applied instead of ultrasonic treatment, but ultrasonic treatment is the simplest method.

In the process of coating the cell membrane vesicles on the porous membrane, the fusion of the cell membrane components can occur to a certain extent even at room temperature, but the optimal condition is about 50 minutes at 50 °C, and depending on the environment, heat treatment can be performed at a certain temperature (for example, 30 °C to 70 °C) for a certain time (for example, 30 minutes to 2 hours). After the heat treatment, the cell membrane vesicles fuse and form layers.

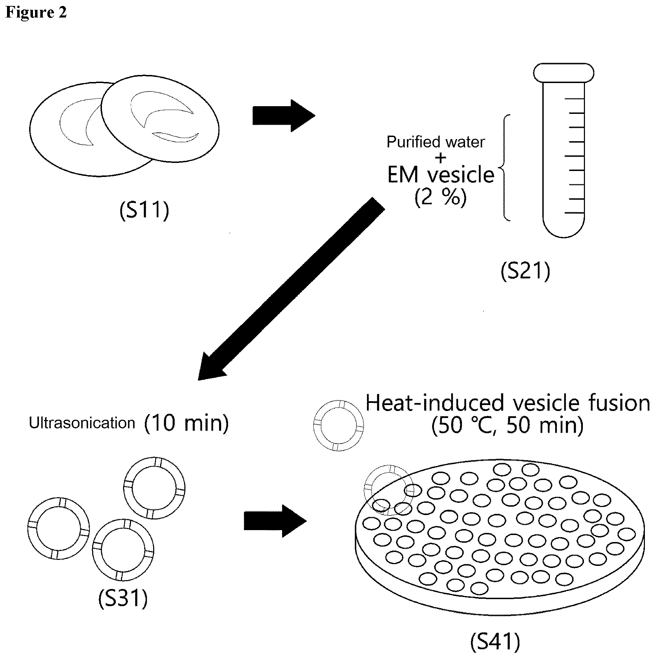

Figure 2 is a diagram illustrating one embodiment of a method for manufacturing a selective concentration filter according to another embodiment of the present invention. The present embodiment is an example in which a red blood cell membrane (erythrocyte membrance) is applied as a selective permeable membrane and anodic aluminum oxide (AAO) is applied as a hydrophilic porous membrane.

The method for manufacturing a selective concentration filter may perform a step (S11) of preparing a red blood cell membrane. The method for manufacturing a selective concentration filter may perform a step (S21) of forming a cell membrane solution by diluting a red blood cell membrane in purified water. The method for manufacturing a selective concentration filter may perform a step (S31) of extracting red blood cell membrane vesicles by ultrasonicating the cell membrane solution for a certain period of time (for example, 10 minutes, etc.). The method for manufacturing a selective concentration filter may perform a step (S41) of pouring red blood cell membrane vesicles on an AAO membrane and heat-treating them at a certain temperature (for example, 30 °C to 70 °C) for a certain period of time (for example, 30 minutes to 2 hours) to form a selective permeable structure in which a cell membrane component-based selective permeable membrane and a porous membrane are laminated.

The AAO membrane is a two-dimensional porous bodies with regular nano-sized pores and is widely used as a membrane, a template, a filter, and the like. As for the porous membrane, any membrane having pores can be used, and a membrane with hydrophilicity helps in water discharge.

In Figures 1 and 2, in the step of forming a cell membrane mixture solution by diluting the cell membrane vesicles in purified water, the cell membrane component in the cell membrane mixture solution is preferably 1 % (v/v) to 4 % (v/v). The cell membrane component may be adjusted within an error range based on 2 %. The error range can be set to 1.9 % to 2.1 %, 1.8 % to 2.2 %, 1.5 % to 2.5 %, or the like, considering the experiment, product, or environment.

The step of pouring the cell membrane solution onto the porous membrane and heat-treating it may be performed at a temperature range of 30 °C to 70 °C. The temperature range and time range can be set differently considering the experiment, product, or environment.

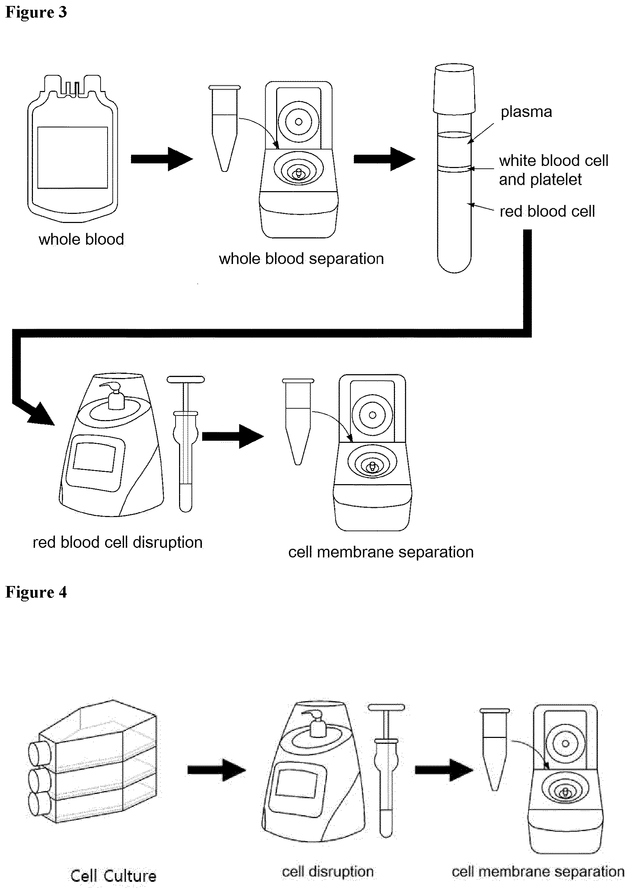

Figures 3 and 4 are diagrams illustrating a step of preparing a cell membrane in a method for manufacturing a selective concentration filter according to one embodiment of the present invention.

Cells (kidney cells, intestinal cells, nerve cells, etc.) are proliferated through cell culture to obtain a sufficient amount, and then the cells are disrupted through a homogenizer or lysis process.

In the case of cells (red blood cells, platelets, white blood cells) present in blood, the blood is separated into three layers: plasma, white blood cells, platelets, and red blood cells through centrifugation, and the layer with the desired cell membrane is separated, and then the cells are disrupted through a homogenizer or lysis process.

Thereafter, anything other than the cell membrane (substances inside the cell, such as hemoglobin in the case of red blood cells) is separated through centrifugation, and the part with the cell membrane is separated.

Referring to Figure 3, the process of extracting the cell membrane (red blood cell membrane) includes the process of first spinning the centrifuge once to remove white blood cells, platelets, plasma, etc., then performing cell disruption through a homogenizer or lysis process, and then spinning the centrifuge again, assuming that it is extracted from whole blood. It is desirable to perform a total of two cycles.

Referring to Figure 4, assuming that other body cells are obtained through culture, the cells are disrupted directly through a homogenizer or the like without using a centrifuge, and then the centrifuge is run. A total of one cycle may be performed.

Once the cell membrane is prepared, the cell membranes in the form of sheets or unspecified forms are made into vesicles through ultrasonic wave to facilitate lamination.

Since the obtained cell membrane (cell membrane component) has a high concentration, it cannot be directly coated on a porous membrane, and it is necessary to control the concentration that can operate as a selective filter.

According to another embodiment of the present invention, a red blood cell membrane may be manufactured according to the following method. In the present embodiment, the cell membrane manufactured according to the following method is referred to as BEETLES[2] membrane.

First, RBCMs (red blood cell membranes) were extracted from blood (anticoagulated human whole blood) stored in K2EDTA to prevent coagulation, and the plasma and buffy coat were removed by centrifugation at 800g for 5 minutes to separate red blood cells. After removing the supernatant, ice-cold 1 X PBS was added to the remaining red blood cells, mixed appropriately, and the washing process under the same conditions was performed three times. Next, the red blood cells were suspended in cold 0.25× for 30 minutes to hemolyze the red blood cells. Next, free hemoglobin was removed in a 20,000g centrifuge for 30 minutes. After repeating this washing process three times, a bright pink RBCMs pellet was obtained, which was then stored at -80 °C.

The extracted RBCMs thus obtained were in the form of negatively charged ribosomes with a size of 200 nm. The ultrasonicated RBCMs were dropped onto an AAO membrane (6809-6002, Whatman, UK) and incubated at 50 °C for 50 minutes to form multiple supported lipid bilayers. The microstructure of RBCMs-AAO was confirmed using SEM, AFM, and fluorescence microscope.

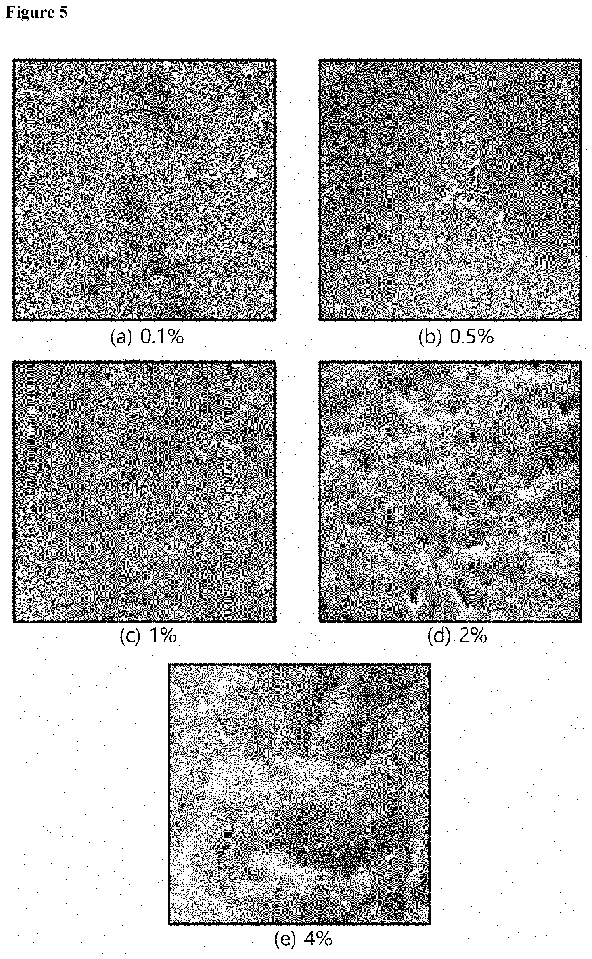

Figures 5 to 9 are diagrams illustrating a surface according to the concentration of a selective concentration filter produced by a method for manufacturing a selective concentration filter according to one embodiment of the present invention.

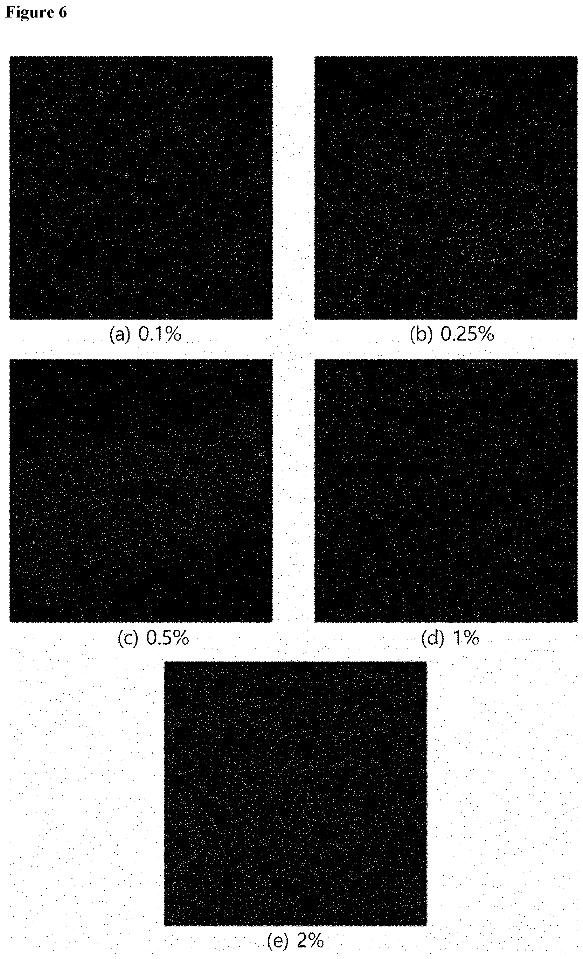

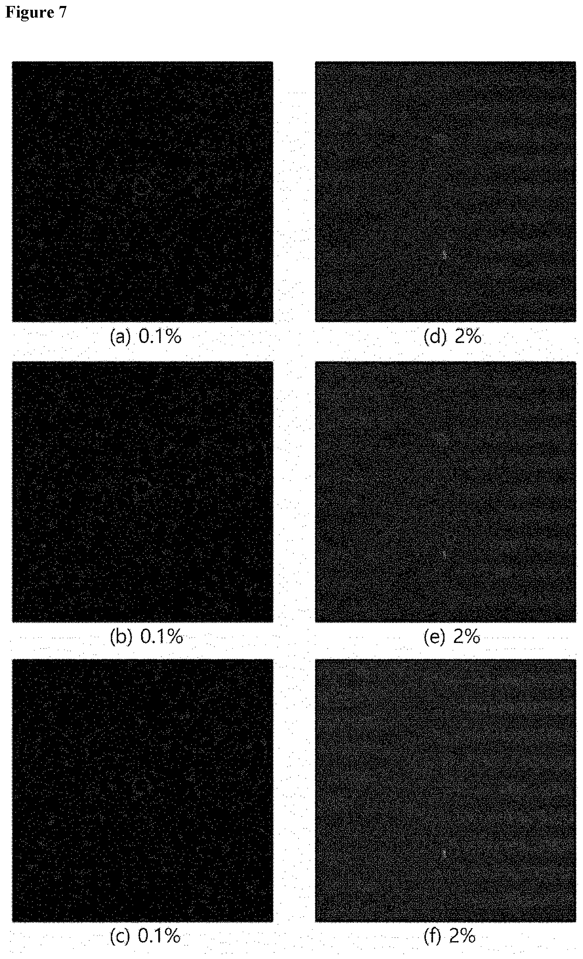

Figure 5 shows an SEM (scanning electron microscope) image of the surface according to the concentration. Figures 6 and 7 show fluorescence analysis images of the surface according to the concentration.

The cell membrane coating using fluorescence can be verified by setting the conditions to fluorescence intensity, gain 600, and pinhole 0.2 %. By tagging the cell membrane with fluorescence, it can be confirmed whether the coating is well done by coating it on the AAO membrane according to the concentration.

A defect can be observed on the surface at a concentration of 0.1%, many vesicles can be observed overall on the surface at a concentration of 0.25%, and it can be seen that the coating is done overall on the surface at a concentration of 0.5%.

Almost no defects are seen on the cell membrane (for example, red blood cell membrane) at the concentrations of 1% and 2 %, which are currently used. On the surface at a concentration of 2 %, the red blood cell membrane is evenly spread overall. It can be estimated that it corresponds to tens to hundreds of layers.

Since the cell membrane is fluid, it is possible to check whether the cell membrane is well coated through the FRAP experiment. The principle is to induce photobleaching by shooting a laser beam with extremely high energy to a certain area, and then check the recovery process to check the degree of coating of the cell membrane.

(a), (b), and (c) of Figure 7 show the results of FRAP (fluorescence recovery after photobleaching) for the cell membrane at a concentration of 0.1%, and (d), (e), and (f) of Figure 7 show the results of FRAP (fluorescence recovery after photobleaching) for the cell membrane at a concentration of 2 %.

(a) and (d) of Figure 7 show the state before photobleaching, (b) and (e) of Figure 7 show the state immediately after photobleaching, and (c) and (f) of Figure 7 show the state after 100 seconds. The results of FRAP experiments on a permeable structure in which a cell membrane is formed at a concentration of 0.1% show that the recovery of the photobleached region is slow. This means that there is no overall fluorescence. The results of FRAP experiments on a permeable structure in which a cell membrane is formed at a concentration of 2 % show that the recovery of the photobleached region is fast, within 100 seconds. This means that the self-recovery ability is very excellent.

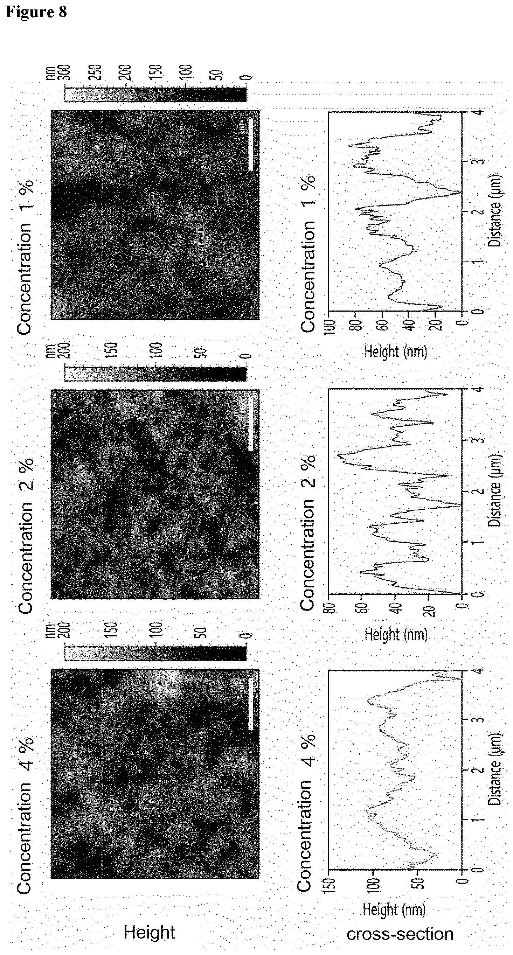

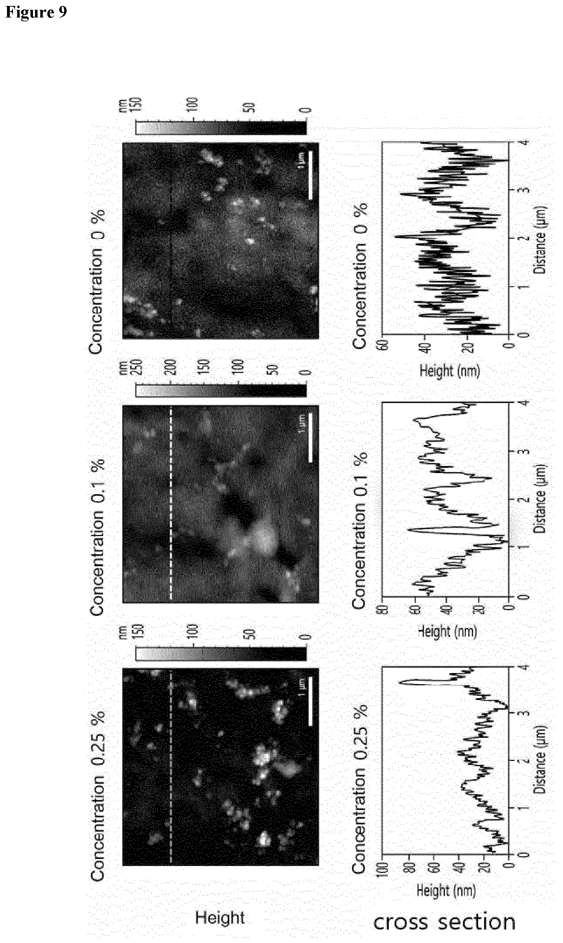

Figures 8 and 9 show AFM (atomic force microscope) images and cross-sections of the surface according to the concentration. In the bare AAO membrane (0%), the AFM is measured while fluctuating due to the pores of the AAO membrane, but as the cell membrane is gradually coated, the pores are covered, reducing the fluctuation, and the coating of the cell membrane can be confirmed in the morphological aspect. From a concentration of 2 % or higher, it shows a very similar appearance, and it can be confirmed that the 2 % concentration is the most optimized.

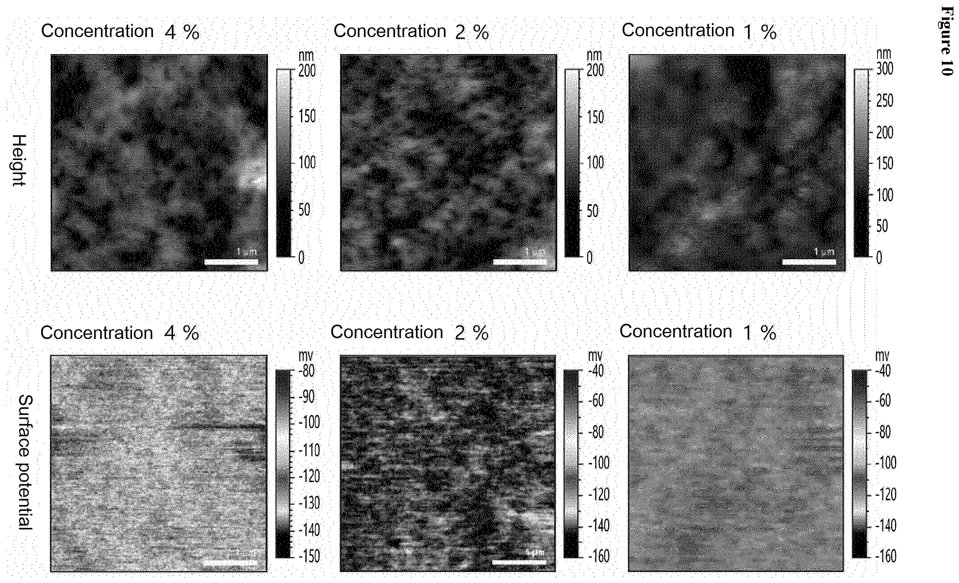

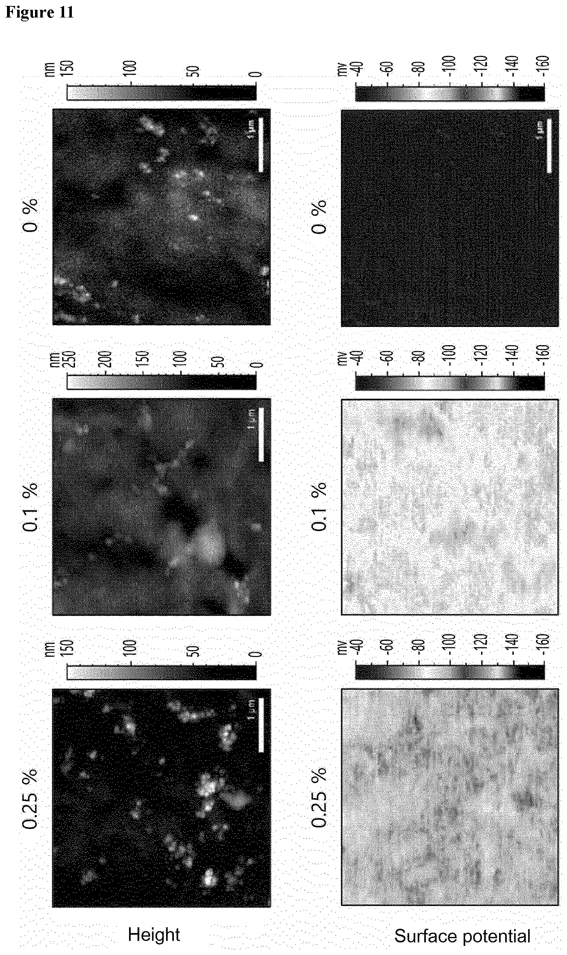

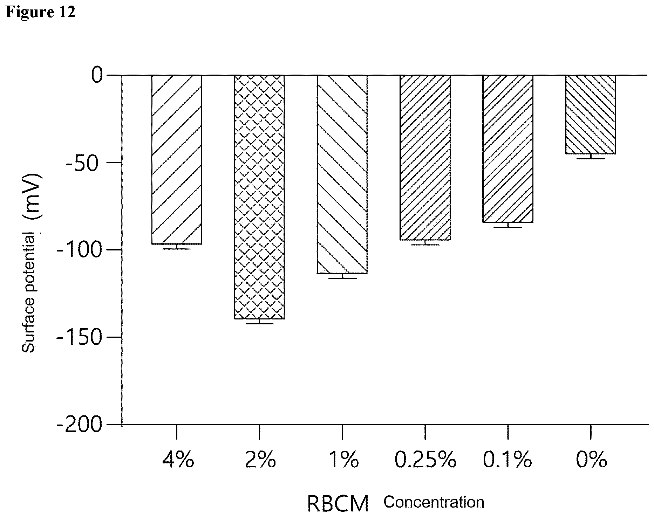

Figures 10 and 11 are diagrams illustrating a surface potential according to the concentration of a selective concentration filter produced by a method for manufacturing a selective concentration filter according to one embodiment of the present invention, and show a KPFM (Kelvin probe force microscope) image and surface potential for the surface according to the concentration. Figure 12 shows surface potential according to the concentration. It can be confirmed that the surface potential of the AAO membrane decreases as the negatively charged red blood cell membrane is coated. The surface potential of the porous membrane changes due to the difference in concentration of the charged cell membrane.

It can be confirmed that the tendency of the surface charge increases in the opposite direction when coating at a concentration of 2 % or more. The change in surface potential of the porous membrane has an inflection at the reference concentration of the charged cell membrane. As the cell membrane is coated thickly, it has dielectric properties, and since it is difficult to measure the surface charge through current based on the principle of KPFM, it can increase. A concentration of 2 % is the most optimized value and is the value at which concentration is performed well. If it is lower than 2 %, the concentration does not meet the standard, and if it is higher than 2 %, the water permeability decreases and the concentration time increases. The concentration range may be adjusted within an error range based on 2 %. The error range can be set to 1.9 % to 2.1 %, 1.8 % to 2.2 %, 1.5 % to 2.5 %, or the like, considering the experiment, product, or environment.

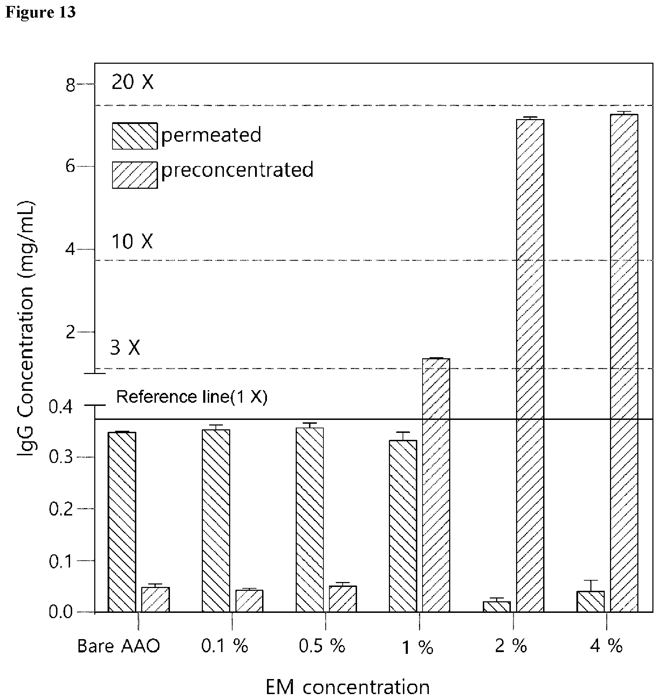

Figure 13 is a diagram illustrating a concentration ratio according to the concentration of a selective concentration filter produced by a method for manufacturing a selective concentration filter according to one embodiment of the present invention. The experiment was performed under the following conditions: a sample volume of 3 mL, a pressure of 3 bar, and a sample buffer of 1 X PBS for IgG. It can be confirmed that the concentration ratio increases rapidly at a concentration of 2 % when the concentration of the cell membrane formed on the porous membrane is increased.

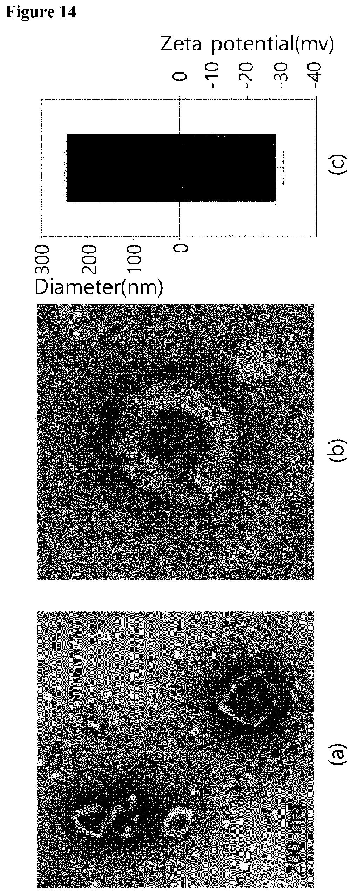

Figure 14 shows an image of the red blood cell membrane in which the cell membrane vesicles are formed according to one embodiment of the present invention. (a) and (b) of Figure 14 show cell membrane vesicles according to different magnifications, and (c) of Figure 14 shows zeta potential.

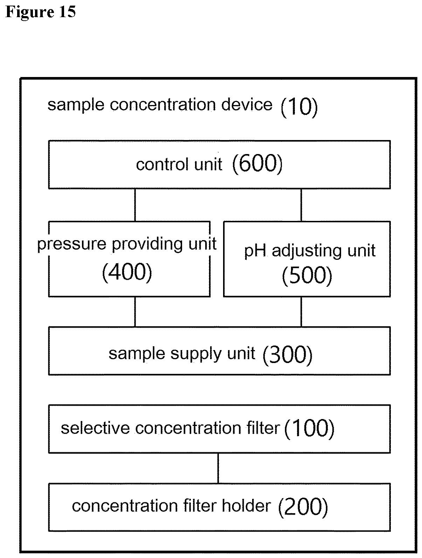

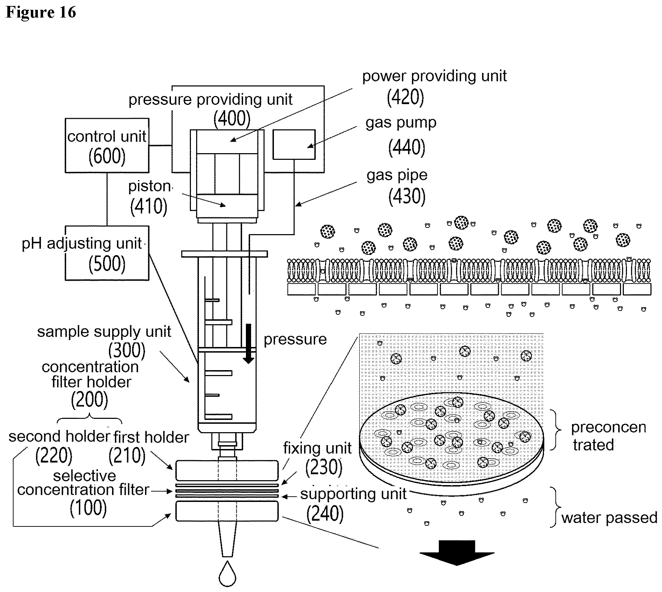

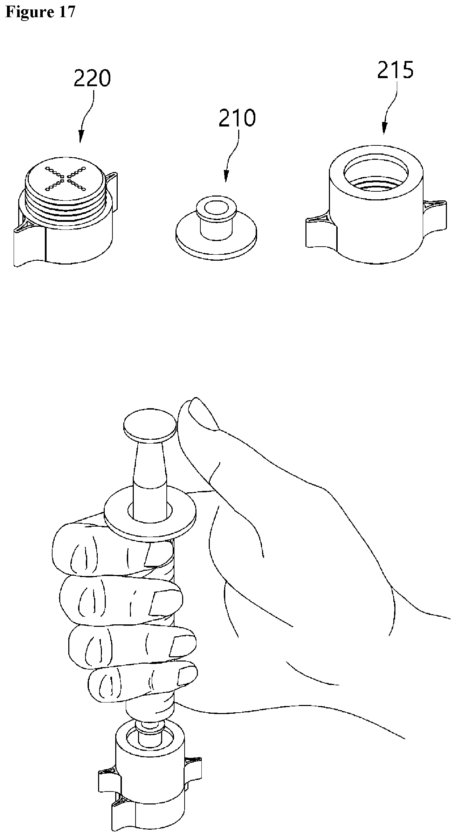

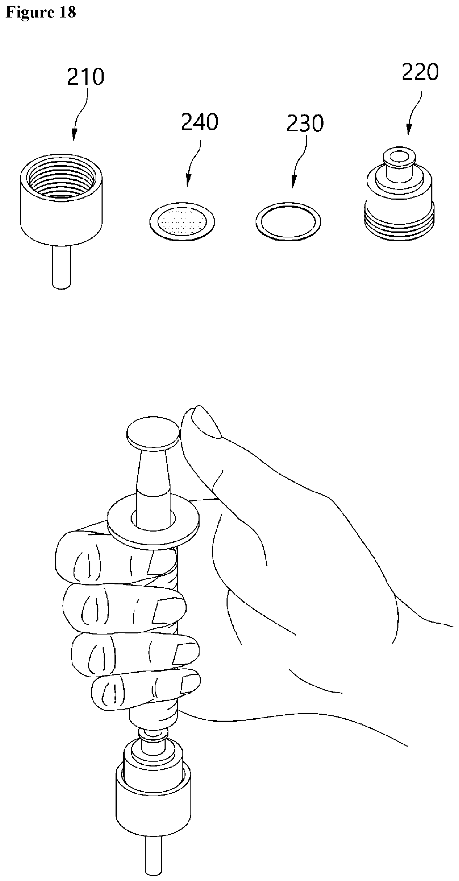

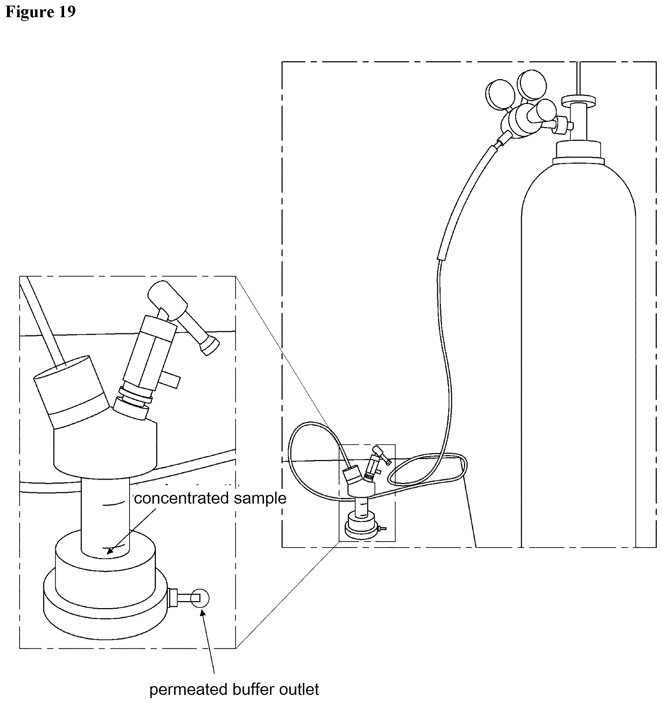

Figures 15 to 19 are diagrams illustrating a sample concentration device according to another embodiment of the present invention.

The sample concentration device (10) comprises a selective concentration filter (100), a concentration filter holder (200), and a sample supply unit (300). The selective concentration filter (100) is formed as a selective permeable structure in which a selective permeable membrane and a porous membrane are laminated. The concentration filter holder (200) surrounds the selective concentration filter and has a structure in which an inlet and an outlet are formed. The sample supply unit (300) is connected to the inlet of the concentration filter holder and has a space for storing a sample. The sample supply unit (300) can be formed in the shape of a syringe, and other shapes are also possible as long as it has a structure in which pressure can be applied. The syringe shape can be sufficiently operated with the strength of a finger.

The sample concentration device (10) may further comprise a pressure providing unit (400) that provides pressure to the sample stored in the sample supply unit by pressing the sample by itself or supplying gas.

The pressure providing unit (400) may comprise a piston (410) for pressing the sample by itself and a power providing unit (420) for providing power to the piston. It may also comprise a pressure measuring unit for measuring the pressure provided.

The pressure providing unit (400) may comprise a gas pipe (430) for supplying gas and a gas pump (440) connected to the gas pipe. It may also comprise a gas measuring unit for measuring the gas provided.

The pressure providing unit (400) can apply the stirred cell operating principle to provide a constant pressure. When pressure is applied with nitrogen gas, only the buffer comes out and only the concentrated sample remains on the selective permeable structure.

The sample concentration device (10) may further comprise a pH adjusting unit (500) connected to the control unit to adjust the hydrogen ion concentration (pH) by adding ions to the buffer solution contained in the sample stored in the sample supply unit.

The sample concentration device (10) may further comprise a control unit (600) connected to the pressure providing unit to control the range of pressure provided by the pressure providing unit.

The selective concentration filter (100) forms a migration path through which some components of the sample pass through the selective permeable membrane (120) and the porous membrane (110), and some components of the sample may move from the sample supply unit to the inlet, move from the inlet to the selective concentration filter, and move from the selective concentration filter to the outlet by pressure.

The concentration filter holder (200) comprises a first holder (210) at the inlet and a second holder (220) at the outlet, and the concentration filter holder may form a structure that can be mutually fastened through the first holder (210) and the second holder (220) or may comprise a separate holder fastening unit (215).

The concentration filter holder (200) may position the selective concentration filter (100) between the first holder (210) and the second holder (220).

The concentration filter holder may comprise a fixing unit (230) that seals the selective concentration filter and a supporting unit (240) that supports the selective concentration filter against pressure, which are located between the first holder and the second holder. The fixing unit (230) may be implemented as a rubber ring or the like, and the supporting unit (240) may be formed as a mesh structure.

The sample comprises an analyte and a fixed separation substance. The sample may include an analyte, a fixed separation substance, and a dynamic separation substance. For example, the fixed separation substance may be water, and the dynamic separation substance may be a specific protein.

The control unit (600) may store a property-concentration data table that records (i) a first property regarding the size of the analyte, (ii) a second property regarding a positive or negative charge of the analyte, and (iii) a third property regarding hydrophobicity or hydrophilicity of the analyte.

The control unit (600) may transmit a command to control the pressure providing unit to regulate whether to concentrate the analyte using the first property, the second property, the third property, or a combination thereof based on the property-concentration data table.

The control unit (600) may store a pressure-permeation data table that records a first pressure section or a second pressure section distinguished according to a reference pressure.

The control unit (600) may transmit a command to the pressure providing unit to control the pressure providing unit to regulate the degree of permeation of the dynamic separation substance in the first pressure section or the second pressure section based on the pressure-permeation data table.

The selective concentration filter of the sample concentration device performs the removal of the fixed separation substance in the first pressure section to perform the concentration of the analyte, and the selective concentration filter can perform the removal of the fixed separation substance in the second pressure section to perform the concentration of the analyte while simultaneously performing the removal of the dynamic separation substance.

The sample may include a buffer solution, an analyte, a fixed separation substance, and a dynamic separation substance.

The control unit (600) may store a pH-permeation data table that records the first hydrogen ion concentration section or the second hydrogen ion concentration section distinguished according to the reference hydrogen ion concentration.

The control unit (600) may transmit a command to the pH adjusting unit to control the pH adjusting unit to regulate the degree of permeation of the dynamic separation substance in the first hydrogen ion concentration section or the second hydrogen ion concentration section based on the pH-permeation data table.

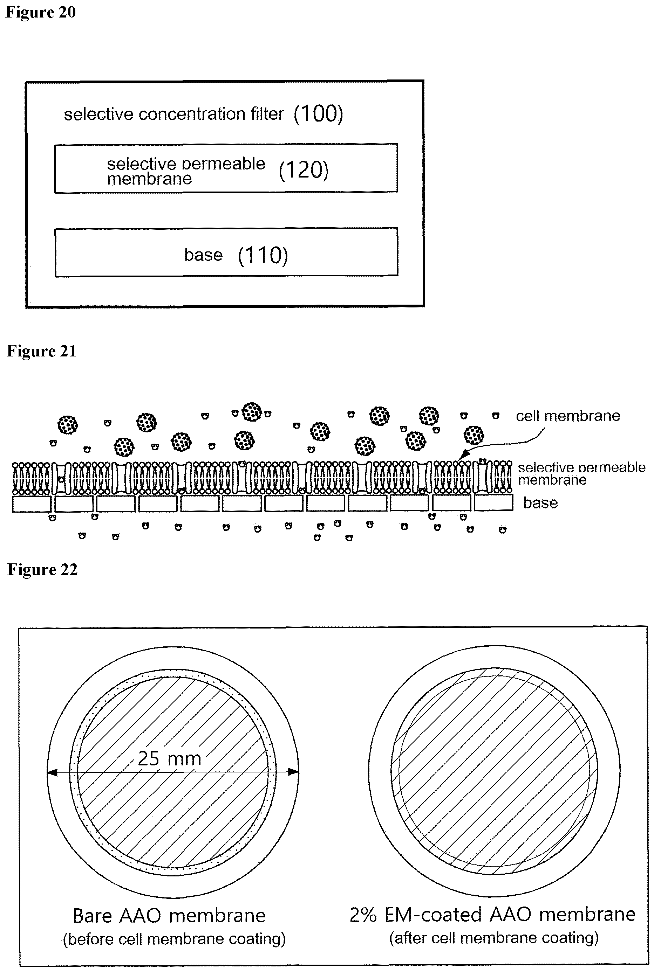

Figures 20 and 21 are diagrams illustrating a selective concentration filter according to another embodiment of the present invention.

The selective concentration filter (100) comprises a porous membrane (110) and a selective permeable membrane (120). The selective permeable membrane (120) is formed on the porous membrane and may be formed based on a cell membrane component. The selective concentration filter (100) may be manufactured using a cell membrane solution and may be optimized by regulating the concentration of the cell membrane.

The selective concentration filter (100) forms a selective permeable structure in which the selective permeable membrane and the porous membrane are laminated.

The selective concentration filter (100) concentrates some components of the sample. The selective permeable membrane does not allow the analyte included in the sample to be permeated, and the selective concentration filter may form a migration path for the fixed separation substance included in the sample to pass through the selective permeable membrane and the porous membrane. For example, the target biomolecule may be concentrated by selectively removing only water from a solution containing a virus.

A pressure (for example, 3 bar) may be applied even with a small force through the highly permeable porous membrane and the selective permeable cell membrane, and the concentration result may be obtained for several minutes (for example, 5 minutes). The concentrated sample may be applied to a commercial LFA (lateral flow immunoassay) kit to improve performance (LOD (limit of detection), sensitivity, etc.).

In an environment where the sample volume is 3 mL, the operation time is 2 minutes, and the pressure difference is 3 bar, the permeability of the bare AAO membrane is 2050 LMH/bar, and the permeability of the AAO membrane coated with the red blood cell membrane is 332.41 LMH/bar. If LMH is 300 or higher, it corresponds to a very high level of flow rate.

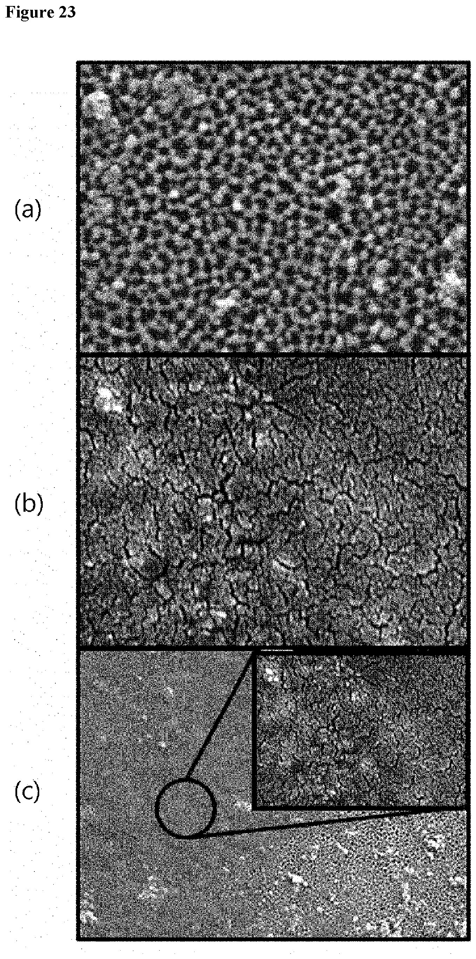

Figures 22 and 23 are diagrams illustrating a surface of a selective concentration filter according to another embodiment of the present invention.

The drawing of Figure 22 is a structure in which a 2 % concentration cell membrane coating is completed on an AAO membrane with a size of 25 mm. When the cell membrane coating is completed, it becomes slightly reddish.

(a) of Figure 23 is an SEM image of a basic porous membrane, and (b) and (c) of Figure 23 are SEM images in which a 2 % concentration cell membrane coating is completed on the porous membrane. It can be confirmed that the surface is evenly coated. The SEM image is an image of a cell membrane coated on an AAO membrane having a pore size of about 20 nm. It can be confirmed that the cell membrane according to the method of the present invention is well coated on the AAO membrane. Therefore, it can be applied to various base layers in addition to AAO. The diameter of the vesicle formed on the selective permeable membrane is in the range of 100 to 300 nm, and the pore diameter of the base is preferably smaller than the diameter of the vesicle. Considering that the diameter of the vesicle-shaped red blood cell membrane in the present embodiment is about 200 nm, the pore size of the membrane is preferably smaller than the diameter of the red blood cell membrane, or is in the range of 10 to 150 nm. This is because if the pore size is too small, it takes a long time to concentrate the analyte, and if the pore size is too large, there is a concern that the red blood cell membrane may be discharged through the pore even at low pressure.

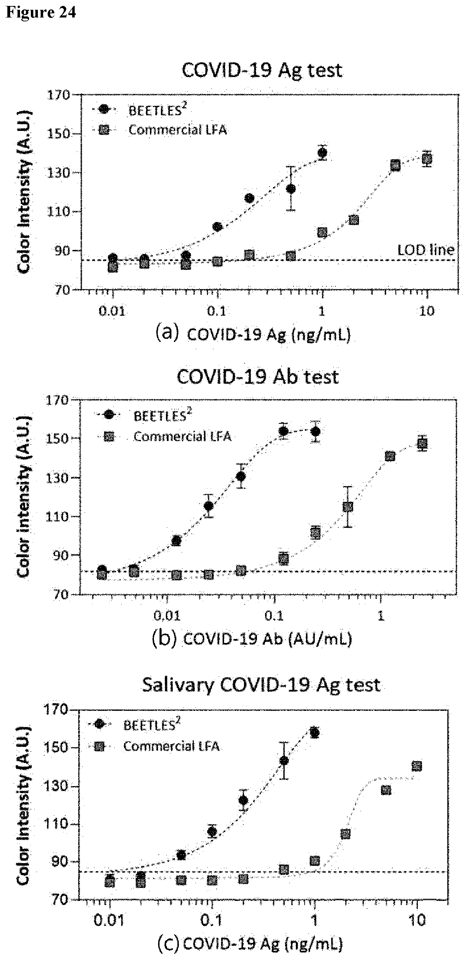

Figures 24 to 28 are diagrams illustrating a concentration ratio of a selective concentration filter according to another embodiment of the present invention.

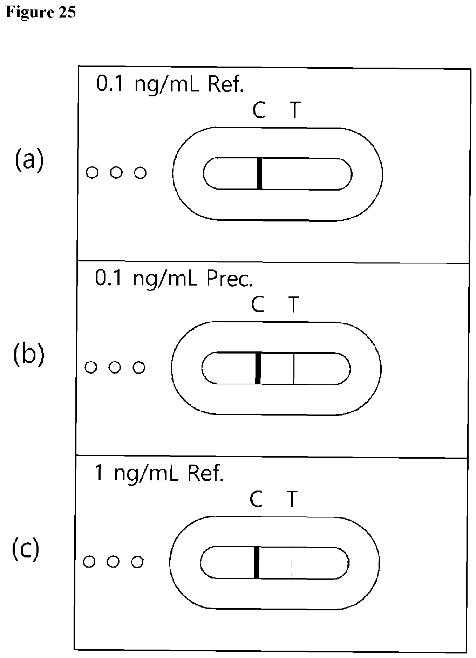

Referring to Figures 24 and 25, the nucleocapsid protein was concentrated under the following conditions: a sample volume of 3 mL, a concentration time of 3 to 6 minutes, and a pressure of 3 bar. In Figure 24, (a) shows the COVID-19 Ag test result, (b) shows the COVID-19 IgG rapid test result, and (c) shows the COVID-19 Ag test result in artificial saliva. VTM (virus transport medium); SDS-PAGE, sodium dodecyl sulfate-polyacrylamide gel electrophoresis; BEETLES[2], a bioengineering concentration tool for LFA of the present invention with improved sensitivity and selectivity. Since IgG is also concentrated, it can be seen that the performance of the COVID-19 antibody kit is also improved. In addition, it can be seen that samples containing nucleocapsid protein in artificial saliva can also be concentrated, and that LFA performance is improved.

Comparing the kit results detected with a concentrated concentration in Figure 25 (b) and the kit results detected without concentration in Figure 25 (c), it can be confirmed that a concentration ratio of 20 times or more can be obtained by selective concentration using the cell membrane.

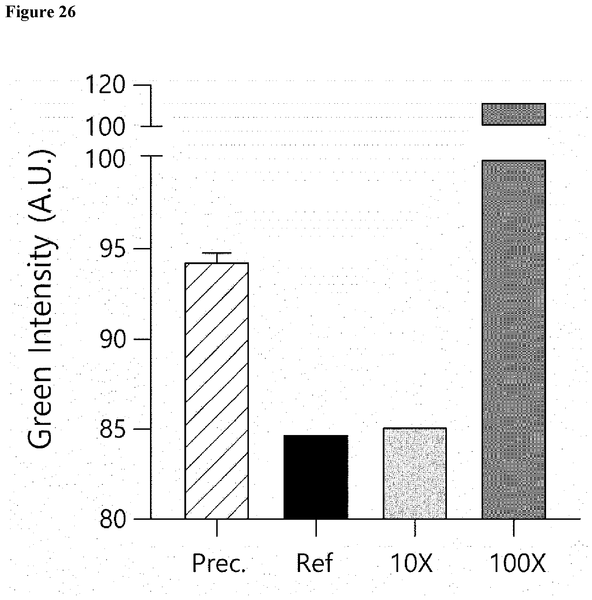

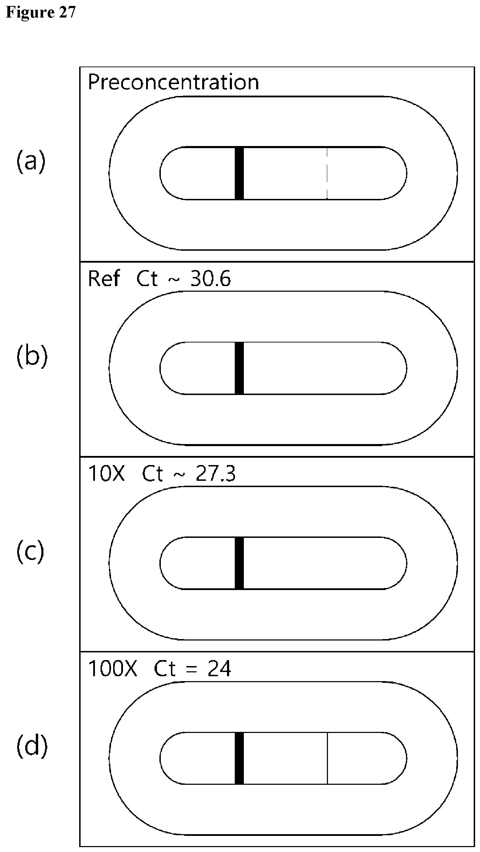

Referring to Figures 26 and 27, a patient sample (Nasal) with a Ct value of 24 was concentrated by diluting it 100 times (Ct to 30.6), and a concentration ratio of at least 20 times or more could be obtained, and it can be confirmed that a patient sample with a Ct value of 30 or more can also be detected by the LFA kit.

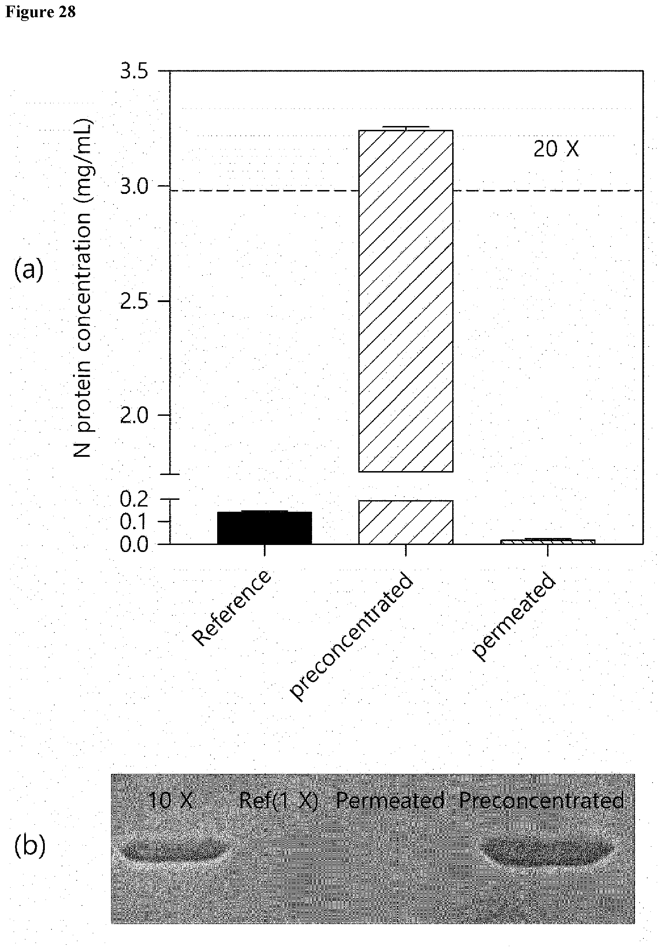

Referring to Figure 28, it can be confirmed that the COVID-19 nucleocapsid protein can be concentrated through the cell membrane-coated porous membrane laminated structure, and that it can be concentrated more than 20 times by cross-verification with nanodrop and SDS-PAGE.

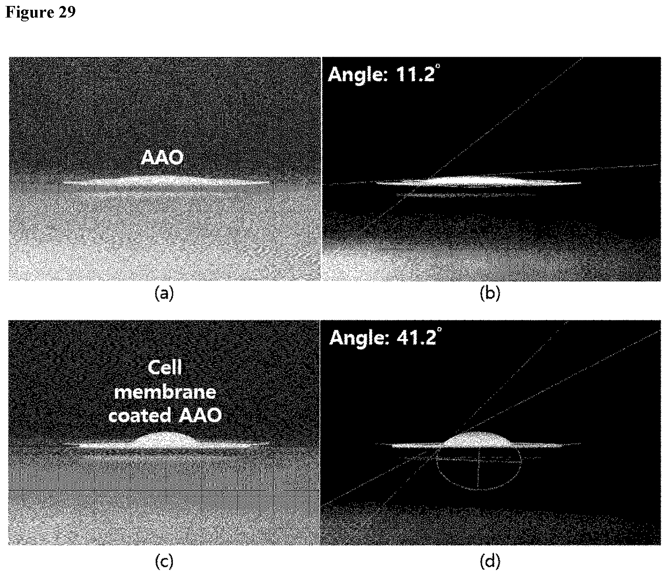

Figure 29 is a diagram illustrating a contact angle of a selective concentration filter according to another embodiment of the present invention. It can be confirmed that the porous membrane maintains hydrophilic properties even after cell membrane coating.

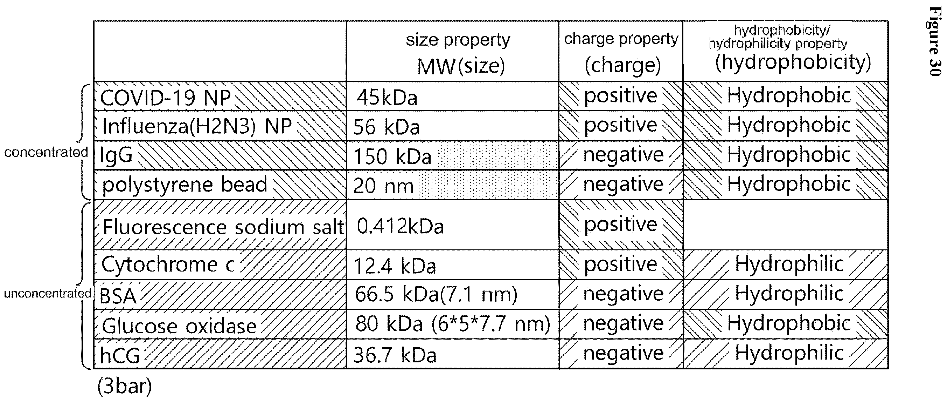

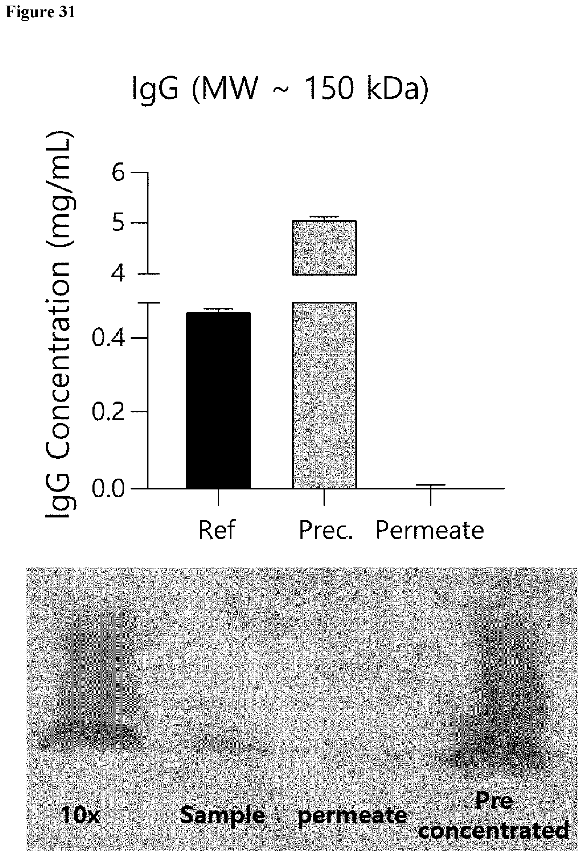

Figures 30 to 32 are diagrams illustrating a concentration ratio of a selective concentration filter according to another embodiment of the present invention that can be adjusted using the sample properties. The concentration characteristics vary depending on the size, charge, and hydrophobicity of the target molecule.

The selective concentration filter can regulate whether or not to concentrate the analyte by using the first property (size property) regarding the size of the analyte that is not permeated by the selective permeable membrane and is concentrated.

The selective concentration filter can regulate whether the analyte is concentrated by using the second property (charge property) regarding the positive or negative charge of the analyte that is not permeated by the selective permeable membrane and is concentrated. The selective concentration filter can regulate whether the analyte is concentrated by using the third property (hydrophobicity/hydrophilicity property) regarding the hydrophobicity or hydrophilicity of the analyte that is not permeated by the selective permeable membrane and is concentrated.

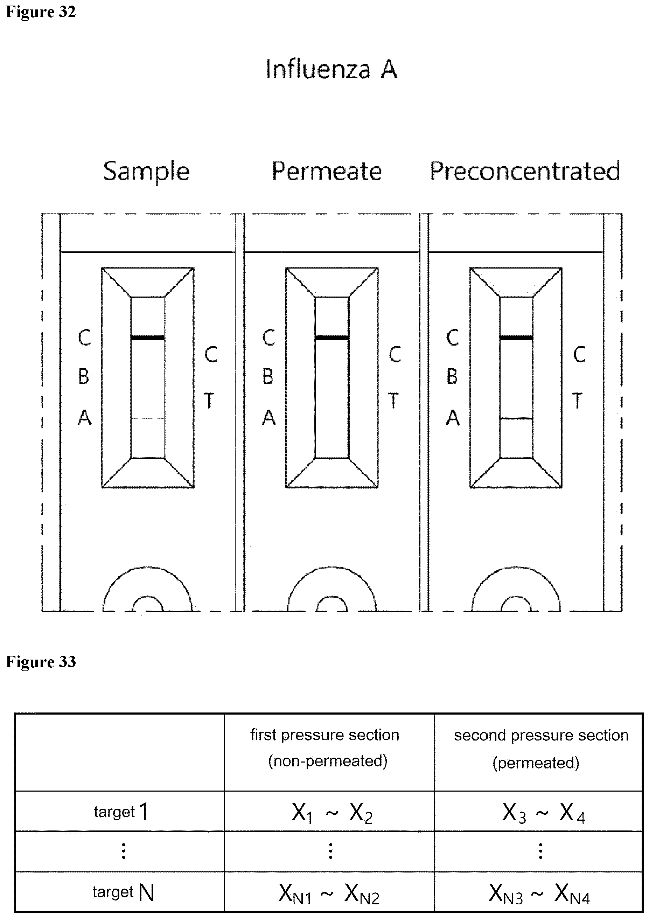

If the molecular weight is 100 kDa or more, it cannot pass through the cell membrane and is concentrated. Referring to Figure 29, if the size property of the target molecule is larger than the reference size, it can be concentrated. For small samples of several tens of kDa, a sample that has a positive charge and is hydrophobic is concentrated. Depending on the size property of the target molecule, even if it is smaller than the reference size, it can be concentrated depending on the charge property and/or the hydrophobicity/hydrophilicity property. Referring to Figure 32, influenza, which is a nucleocapsid protein, can also be concentrated.

A cell surface having hydrophilicity forms a hydration layer, thereby preventing protein adsorption. The negatively charged cell membrane and the positively charged protein are electrically attracted, resulting in weak adsorption. If the surface is relatively hydrophobic, weak adsorption occurs. Therefore, a protein that is both positively charged and hydrophobic cannot pass through the cell membrane and is concentrated.

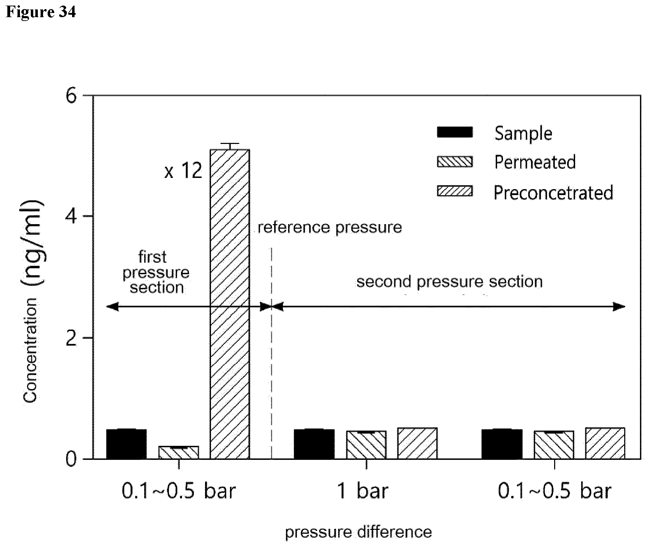

Figures 33 to 38 are diagrams illustrating a concentration ratio that can be adjusted using pressure properties applied to a selective concentration filter according to another embodiment of the present invention.

The sample includes an analyte, a fixed separation substance, and a dynamic separation substance, and the selective permeable membrane can regulate the degree of permeation of the dynamic separation substance by regulating the spacing of the phospholipids of the cell membrane in the first pressure section or the second pressure section distinguished according to the reference pressure.

The selective concentration filter can perform the removal of the fixed separation substance in the first pressure section to perform the concentration of the analyte. The selective concentration filter can perform the removal of the fixed separation substance in the second pressure section to perform the concentration of the analyte while simultaneously performing the removal of the dynamic separation substance. BSA originally does not pass through the cell membrane, but the selective concentration filter can control the permeation of BSA by applying pressure.

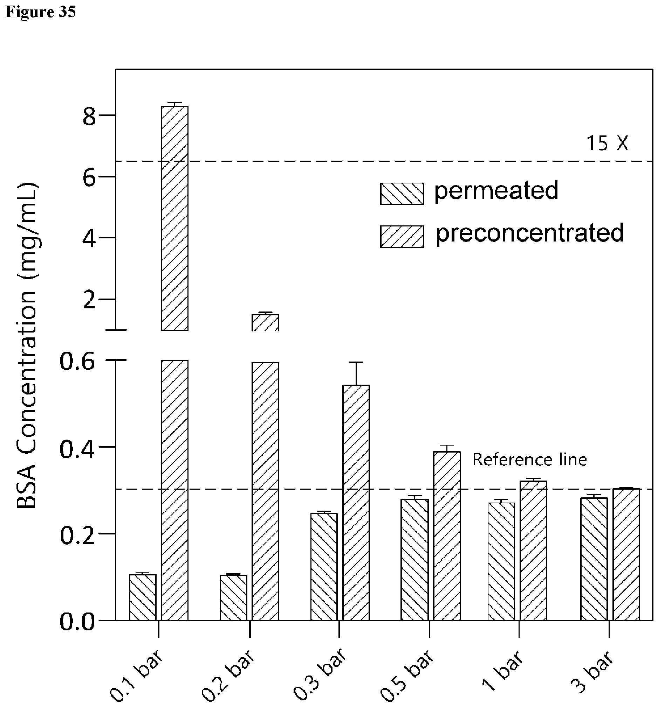

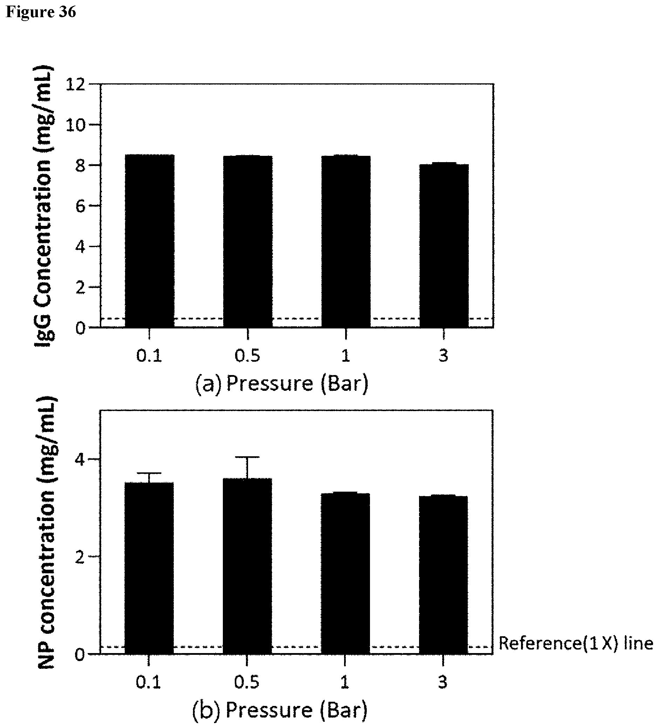

Figure 36 is a graph showing the degree of concentration of IgG and NP according to the applied pressure. As shown in Figures 35 and 36, it can be seen that both IgG and NP are well concentrated, unlike BSA, without being affected by pressure.

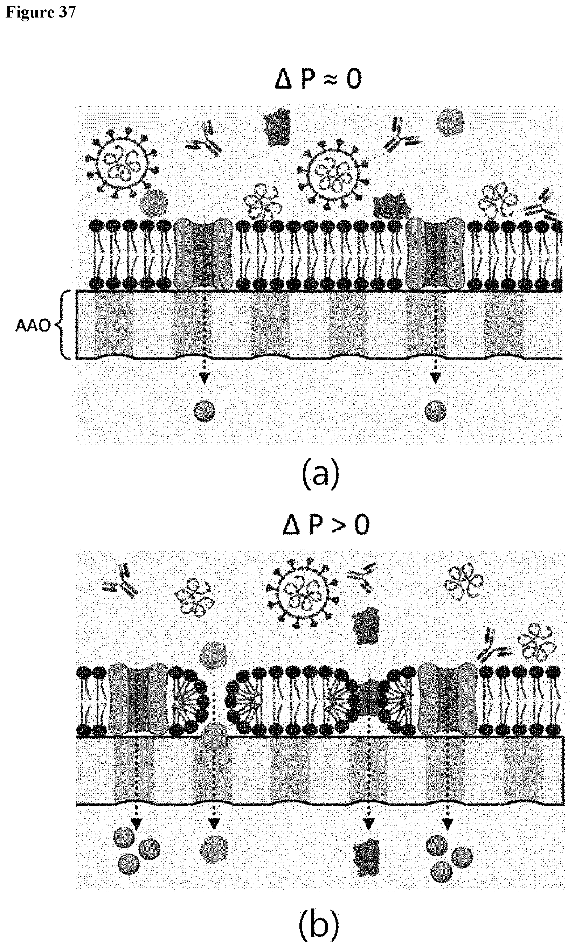

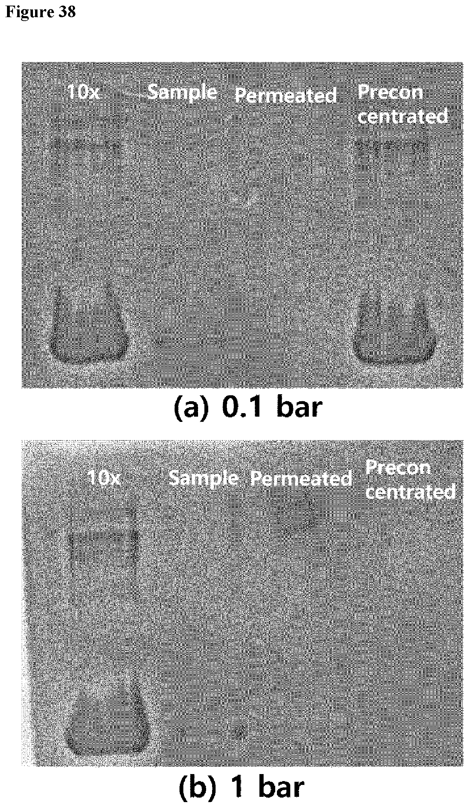

(a) of Figure 37 shows that when the pressure is close to 0, most biomolecules except water are not allowed to be permeated. (b) of Figure 37 shows that when the pressure increases (for example, about 0.5 bar or more), the phospholipids spread apart, allowing proteins (small proteins with negative charges) to pass through the gaps between the phospholipids. Referring to Figure 38, the gaps between the phospholipids in the cell membrane are widened further due to the pressure corresponding to the second pressure section (for example, 1 bar to 3 bar), allowing BSA to pass through, but when the pressure corresponding to the first pressure section (for example, 0.1 bar to 0.5 bar) is less applied, the gap ($d_1$) is not widened sufficiently, preventing BSA from passing through.

BSA theoretically cannot pass through the cell membrane, but the gaps between the phospholipids in the cell membrane are created by applying pressure, allowing it to pass through the cell membrane. By utilizing these characteristics, unnecessary albumin can be selectively removed in the diagnosis of diseases.

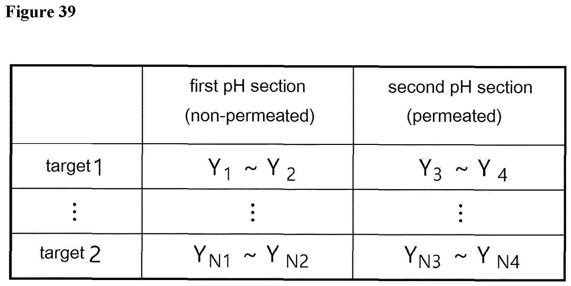

Figures 39 to 41 are diagrams illustrating a concentration ratio of a selective concentration filter according to another embodiment of the present invention that can be adjusted using hydrogen ion concentration properties of a sample buffer.

The sample includes a buffer solution, an analyte, a fixed separation substance, and a dynamic separation substance, and the selective permeable membrane can regulate the degree of permeation of the dynamic separation substance by regulating the hydrogen ion concentration of the buffer solution in the first hydrogen ion concentration section or the second hydrogen ion concentration section, which are distinguished according to the reference hydrogen ion concentration.

The selective concentration filter can perform the removal of the fixed separation substance in the first hydrogen ion concentration section to perform the concentration of the analyte. The selective concentration filter can perform the removal of the fixed separation substance in the second hydrogen ion concentration section to perform the concentration of the analyte while simultaneously performing the removal of the dynamic separation substance.

Since BSA has a pI (isoelectric point) value of approximately 4.9, it has a negative charge in a physiological environment.

Since the cell membrane can concentrate molecules with a positive charge, the concentration can be performed by lowering the pH of the buffer to make BSA positively charged.

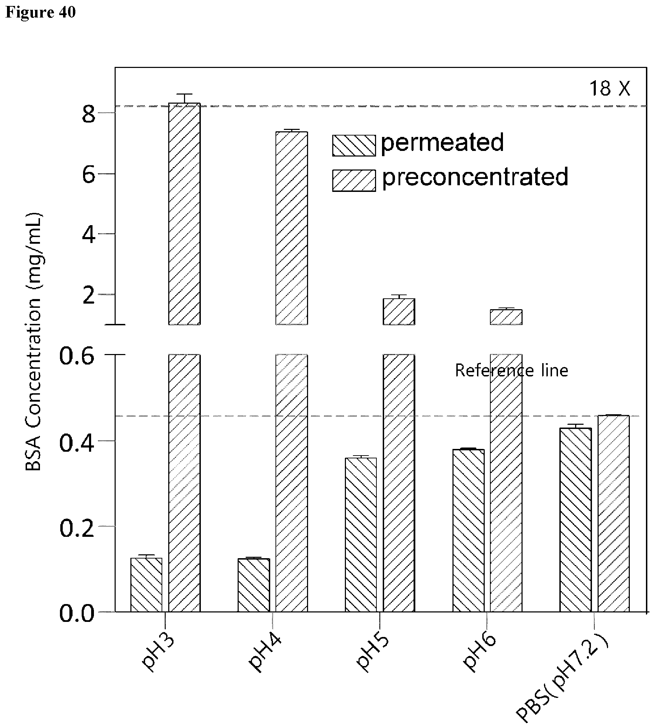

Referring to Figure 40, HCl is added little by little to PBS at a pressure of 3 bar to lower the pH. When BSA becomes positively charged at low pH, it can be confirmed that it does not pass through even when pressure is applied and is concentrated.

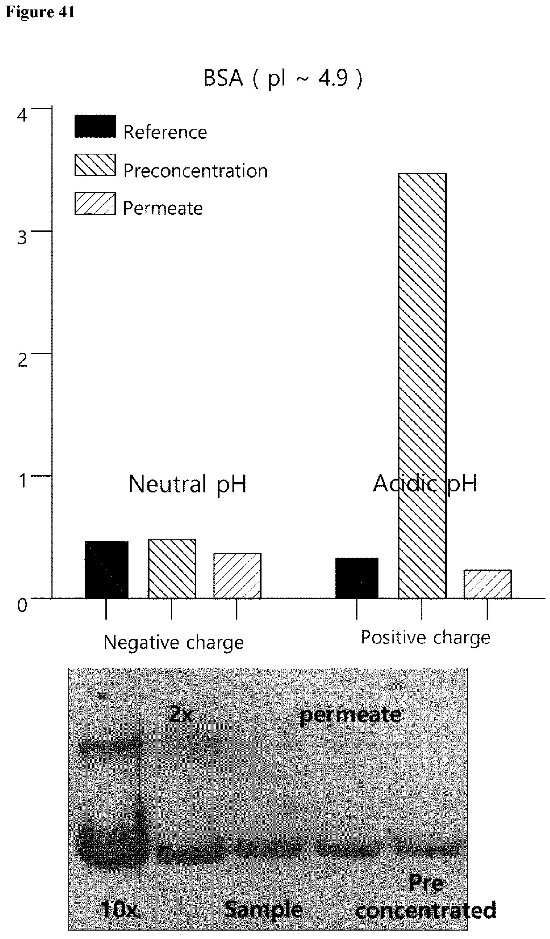

Referring to Figure 41, It can be confirmed that BSA (bovine serum albumin) protein is concentrated because it has an isoelectric point (pI) of 4.9 and has a positive charge due to its acidic nature.

The selective concentration filter according to the present embodiment can regulate cell membrane permeation characteristics according to pressure and charge, and can control concentration and permeation conditions according to pressure and charge properties (pH concentration).

[Example of method for analyzing characteristics of BEETLES[2] membrane]

A method for analyzing characteristics of BEETLES[2] membrane according to one embodiment of the present invention is described in detail. The AAO-RBCM membranes were sampled for KPFM imaging on a p-type silicon wafer (ePAK International, USA), which is an electrically conductive substrate. The silicon wafers were soaked in piranha solution ($H_2O_2$ and $H_2SO_4$) for 15 minutes, washed with distilled water, and dried with $N_2$ gas (Sejong Industry Gas, Republic of Korea). The AAO-RBCM membranes were electrically and topologically analyzed in amplitude-modulated KPFM mode (Bruker, USA) using a MultiMode VIII atomic force microscope. KPFM measurements were performed at 23 °C and in air using a lift-scan mode based on tapping mode. The nanoelectrical properties of the samples were analyzed using a Pt-coated conductive atomic force microscope tip (SCM-PIT-V2; Bruker, USA). Topological AFM images were captured in the first scan using the tapping mode with zero tip bias.

In order to detect the surface potential, the AFM tip was lifted 20 nm above the sample surface with the sample bias voltage applied during the interleaved scan. During the interleaved scan, the mechanical drive to the cantilever was turned off, and an alternating current (AC) bias voltage of 1,000 mV was applied to the probe at the mechanical resonance ($\omega$) of the cantilever. VAC caused the cantilever to oscillate due to the attractive and repulsive electrostatic interactions (Fes) between the probe and the sample.

[Equation 1]

$$F_{es} = -\frac{1}{2}\frac{dC}{dz}[(V_{DC} - V_{CPD}) + V_{AC}sin(\omega t)]^2,$$

where VCPD is the contact potential difference between the probe and the sample, and VDC is the direct current (DC) bias voltage.

A proportional-integral-differential feedback loop monitored and controlled the amplitude of the cantilever oscillation by applying a compensating VDC to the probe to cancel the probe-sample electrostatic force (Fes). This depends on the probe-sample capacitance C and the height z. For KPFM operation, the scan rate is 0.6 Hz, and the scan size and the amplitude settings at 12 nm are 4 $\mu$m $\times$ 4 $\mu$m at 512 $\times$ 512 pixels, respectively. MountainsSPIP software was used for leveling, processing, and analysis of AFM images (version 9, Digital Surf, France). In addition, Gwyddion was used to study the surface roughness (version 2.60).

FRAP was used to verify the SLB formation of RBCM to evaluate the fluidity and diffusivity. In all experiments, 1 wt% PE-CF was inserted into RBCM as a fluorescent probe. A 20 $\mu$m diameter spot in the z-plane of the bilayer was bleached for 5 seconds with a 150 mW, 561 nm optically pumped semiconductor laser (Coherent, Inc.). Recovery of the bleached spots was monitored over 15 minutes using a Zeiss LSM-800 confocal microscope. Each image reference spot was used as a standard to measure and normalize the spot fluorescence intensity. Next, the diffusion coefficient (D) of the dye in each SLB configuration was calculated using $D = w^2/4t^{1/2}$, where w is the radius of the photobleached spot, and $t^{1/2}$ is the time required to achieve half of the maximum recovery of fluorescence intensity.

[Example of reagents and analysis methods]

We tested the improved performance with the commercial COVID-19 Ag Rapid Kit (Calth Inc., Republic of

Korea), BEETLES[2]. The sensitivity and LOD were tested using 1x PBS buffer (LB004, DUKSAN, Republic of Korea) with the COVID-19 N protein recombinant antigen (45 kDa, FPZ0516, Fapon Biotech Inc., China), known as the best COVID-19 Ag test target. Bovine serum albumin (BSA) (A7030, Sigma-Aldrich, USA) and IgG (I4506, Sigma-Aldrich, USA) were purchased from Sigma-Aldrich.

Next, 7 concentrations of COVID-19 N protein samples were prepared with 1x PBS. About 3 mL of each sample was dropped into a polycarbonate stir cell (341000, STERLITECH, USA), and the reaction was allowed to proceed for 3 minutes. Then, the concentrated sample was pipetted into a 100 uL volume. Proteins were quantified using a NanoDrop spectrophotometer (Thermo Scientific, USA) and SDS-PAGE (KOMA precast gel BC type, Koma Biotech, Republic of Korea).

We analyzed the color intensity of commercial LFA kits and BEETLES[2]-supported LFA using LabVIEW software (National Instruments Co., USA) and a custom National Instruments (NI)-controlled optical system.

In order to set the LOD value (Figure 24) and cutoff value, we first measured the color signal using a commercial reader and a custom NI-controlled optical system with LabVIEW software. Next, five individually trained engineers (Calth Inc. http://www.thecalth.com) observed the colorimetric signal using a standard color chart and the manufacturer's instructions to determine the LOD. The cutoff value was set according to the manufacturer's guidelines to minimize false positives for higher specificity.

[Analysis: Clinical Study]

We collected clinical samples of nasopharynx/oropharynx (NP/OP) and saliva from COVID-19 patients at Seoul St. Mary's Hospital with the approval of an institutional review board (KC21TIDI0134K). While NP/OP swabs were contained in viral transport medium (VTM), saliva samples were collected in sterile tubes and diluted with phosphate buffered saline. In order to test the performance on low viral targets, OP/NP samples were spiked in PBS buffer for high viral load (Ct < 25), and then prepared as low viral samples. For saliva samples, samples were spiked in PBS buffer. Finally, low viral samples were prepared with Ct > 30. For healthy controls, samples were collected (or purchased) from healthy volunteers and stored in a -20 °C freezer. The results obtained were compared with the RT-qPCR SARS-CoV-2 test results using the AccuPower SARS-CoV-2 Variants ID2 kit (BIONEER, Republic of Korea) according to the manufacturer's protocol. The combined sensitivity and specificity were maximized to optimize the on-site COVID-19 diagnostic threshold.

In order to implement a prototype for POCT sample preparation, a portable device integrated with the BEETLES[2] membrane was fabricated. We designed two reservoirs: a sample reservoir and a commercially available buffer reservoir. Micromilling technology was applied to the prototype. We calculated the average hand force pressure applied by five individuals (three men and two women) at $5 \pm 1$ bar.

The hand-pressurized portable syringe can be directly applied to commercial LFA as a low-cost alternative for point-of-care diagnostics. First, the BEETLES[2] membrane was connected to the sample reservoir and the plunger was pressed for concentration. After 3 minutes of operation, the BEETLES[2] membrane was connected in reverse to the running buffer reservoir. Then, it was analyzed using a commercial LFA according to the manufacturer's instructions. A second, more simplified prototype that operates on pressure drive from a vacuum chamber is also possible.

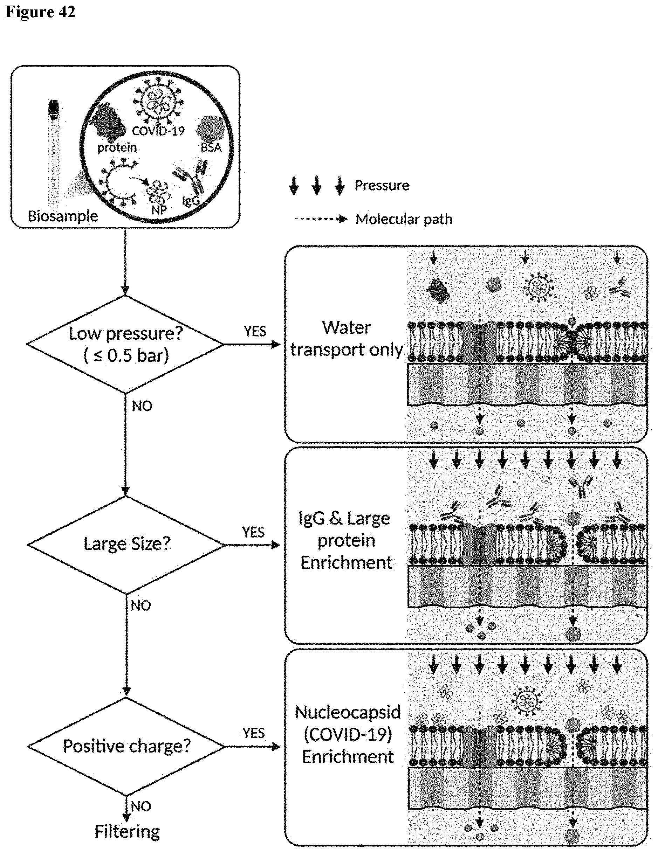

Figure 42 is a flow chart illustrating the concentration characteristics and permeation characteristics of a cell membrane according to pressure, size, and charge. The inserted drawing shows the permeation characteristics of the cell membrane for the COVID-19 virus and several proteins. First, when the pressure is lower than 0.5 bar, a buffer solution such as water can pass through the pores. Even if the pressure is high, if the protein is large, it cannot pass through even if the phospholipids are separated, and as a result, it is concentrated on the base surface.

Of course, small substances escape through the gaps in the phospholipids that are created by the applied pressure, and at this time, positively charged proteins electrostatically stick to the negatively charged phospholipid surface and cannot pass through, and as a result, they are concentrated on the cell membrane surface.

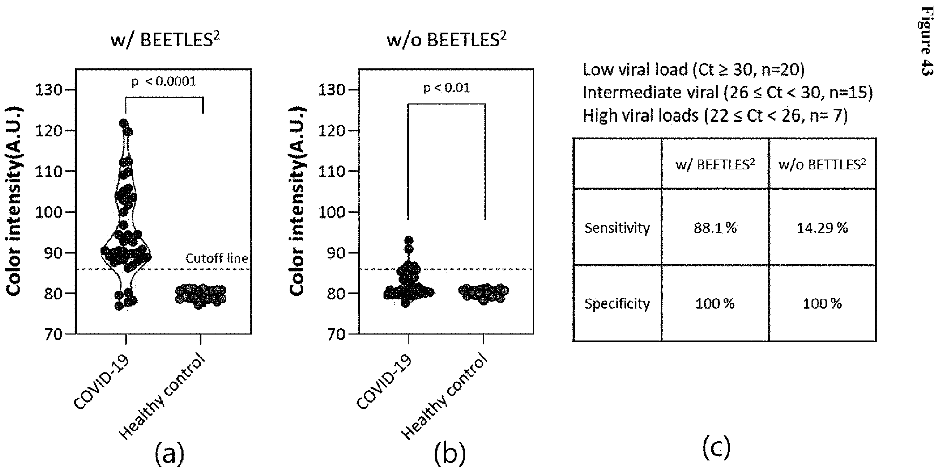

Figure 43 illustrates the results obtained by comparing the sensitivity between the preprocessing system of BEETLES[2] according to one embodiment of the present invention and the existing LFA. The clinical samples include COVID-19 patients (n = 42) and healthy controls (n = 20). In the case of low viral load, in order to observe the improvement, low virus patient samples (Ct $\geq$ 30, n = 20: 48%) were included. All samples of NP/OP, saliva, asymptomatic, and various variants were included. Regardless of the specimen (nasopharyngeal, oropharyngeal, saliva), variant (Delta, Omicron), and asymptomatic patient, we could confirm the performance improvement of the LFA kit (immunodiagnosis) through preprocessing. The sensitivity of the existing LFA was 14.29%, but it can be seen that it was improved to 88.1% through the preprocessing system of BETTLES[2].

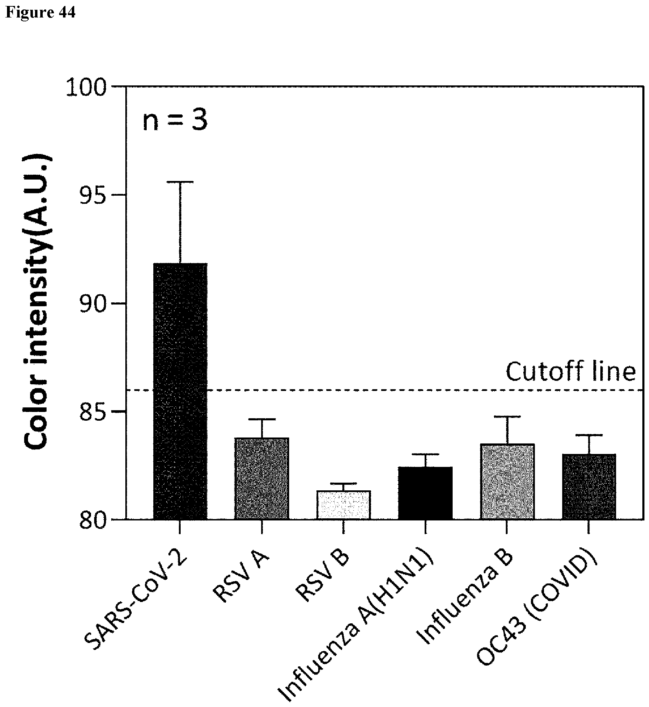

Figure 44 is a reference diagram illustrating the results of the selectivity test of LFA. Referring to Figure 44, it can be seen that when viruses other than SARS-CoV-2 were preprocessed, no positive reactions were expressed in the LFA kit.

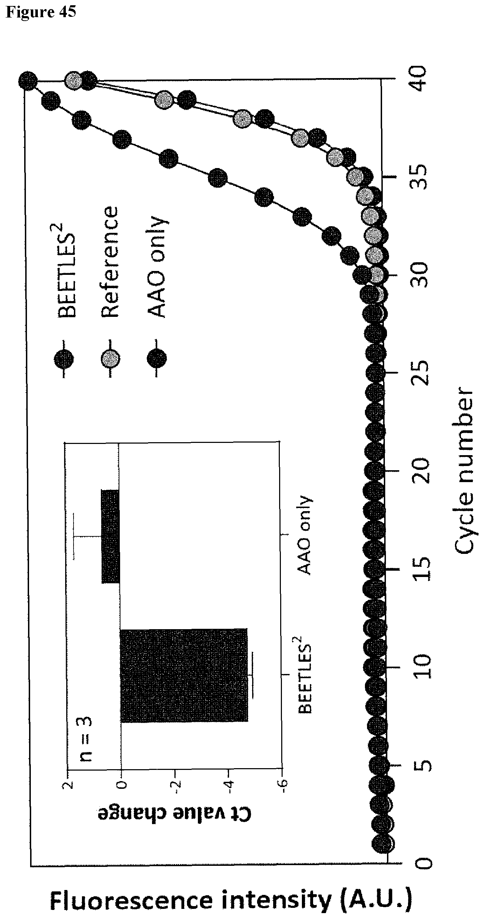

Figure 45 illustrates the results of the molecular diagnosis application (RT-PCR). This is the result obtained by comparing two types of patient samples stored in VTM: AAO membrane (pore size of 20 nm) and AAO membrane coated

with cell membrane (BEETLES[2]). Since the patient samples stored in VTM are likely to be lysed, they are more likely to pass through the pores of the AAO membrane, and therefore, they are not concentrated, so it can be confirmed that the Ct value is almost unchanged compared to the reference. In the case of BEETLES[2], it can be seen that even lysed viruses can be concentrated, so the Ct value can be lowered to about 5 compared to the reference.

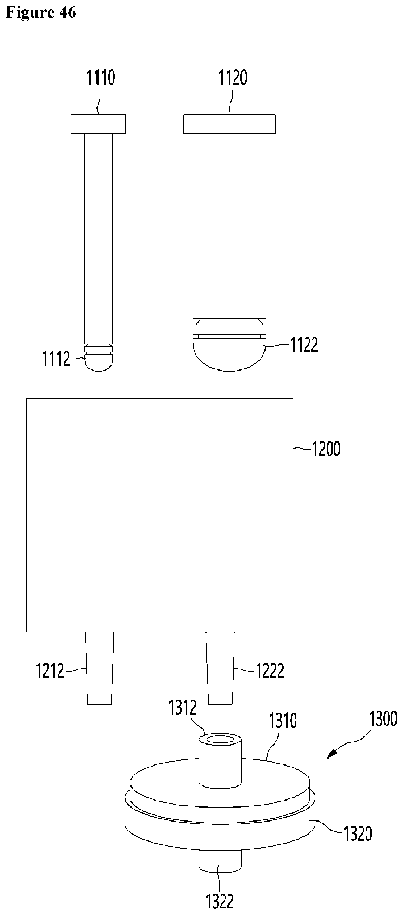

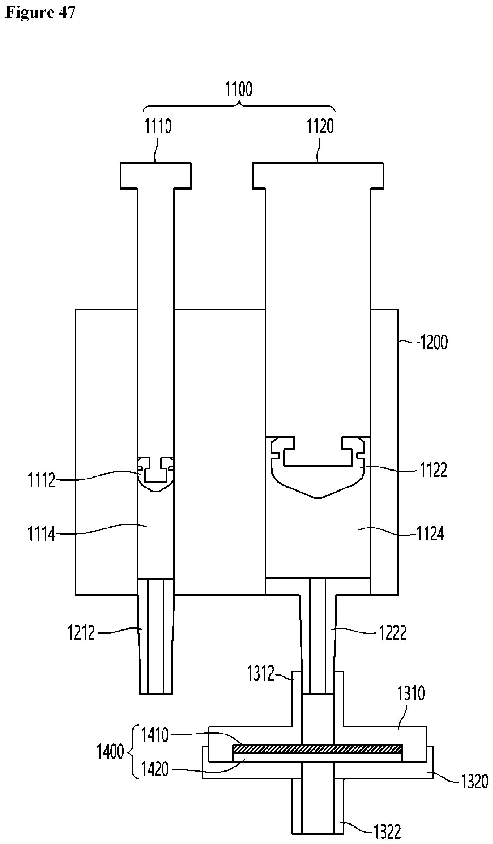

Figure 46 is a configuration diagram illustrating a sample concentration device according to another embodiment of the present invention. The sample concentration device illustrated in Figure 46 comprises a first pressure providing unit (1120), a second pressure providing unit (1110), a body (1200), and a selective concentration unit. Figure 47 is a cross-sectional diagram of a sample concentration device of Figure 46.

Referring to Figures 46 and 47, the selective concentration unit comprises a selective concentration filter (1400) and a concentration filter holder (1300). The selective concentration unit is designed to be separated from and connected to the body (1200), and it is obvious that it can be produced and sold separately from the sample concentration device.

The selective concentration filter (1400) comprises a base (1420) and a selective permeable membrane (1410). The base (1420) has a pore structure and can be provided in a sheet shape. The selective permeable membrane (1410) is coated on the base and has a vesicle formed therein. The selective permeable membrane (1410) does not allow an analyte to be concentrated from a sample to be permeated, and allows at least a portion of the remaining substances other than the analyte to be permeated through the pore structure. Here, the analyte may be a virus to be diagnosed, and the remaining substance may be a buffer solution such as water or an electrolyte.

The concentration filter holder (1300) comprises a first holder (1310) and a second holder (1320), and has an internal space formed to surround a selective concentration filter (1400). The first holder (1310) comprises a first passage (1312), and the second holder (1320) comprises a second passage (1322). The first passage (1312) is located on the selective permeable membrane (1410) of the selective concentration filter, and the second passage (1322) is located on the base (1420) of the selective concentration filter. The first passage (1312) is a passage through which a sample is injected by pressure from the first pressure providing unit (1120). Some of the remaining substances among the samples injected through the passage pass through the base (1420) and are discharged to the outside through the second passage (1322). The analytes of the sample that are not discharged to the outside are concentrated in the selective permeable membrane (1410).

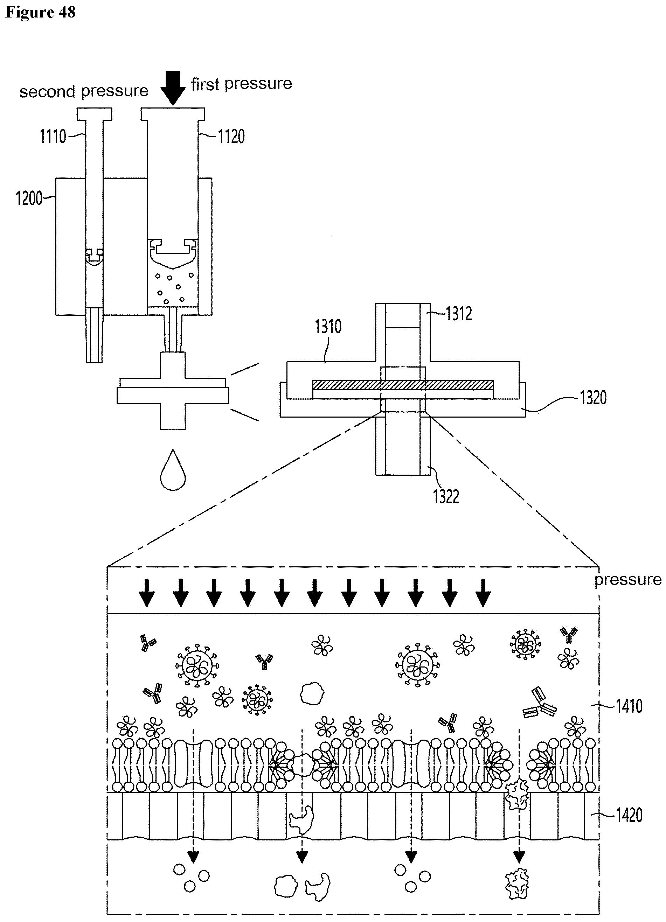

In the present embodiment, the pressure providing unit (1100) comprises a first pressure providing unit (1120) and a second pressure providing unit (1110). The body (1200) supports the pressure providing unit and comprises a path through which the pressure providing unit can move, that is, a channel formed inwardly. Here, the channel may include a first channel (1124) and a second channel (1114). The space of the first channel (1124) is a space for introducing a sample and a buffer solution. When the sample and the buffer solution introduced through the first pressure providing unit (1120) are pressurized, the sample and the buffer solution are transferred to the selective concentration filter (1400) according to the pressurization. Figure 48 is a reference diagram for explaining the concept of concentrating a sample through a sample concentration device. Referring to Figures 47 and 48, a first coupling unit (1222) and a second coupling unit (1212) are formed at the end of the body (1200). The first coupling unit (1222) is provided to be coupled to and separated from the first passage (1322) of the concentration filter housing (1300).

The space of the second channel (1114) is a space where a buffer solution can be selectively introduced, and provides a path for the second pressure providing unit (1110) to move. The selective concentration unit (1300, 1400) in which the sample is concentrated through the first pressure providing unit (1120) is separated from the body (1200) and then recoupled to the body through the second coupling unit (1212). In particular, it is preferable to turn the selective concentration unit over and couple it with the second coupling unit (1212). At this time, the second coupling unit (1212) is coupled with the second passage (1322). According to the pressurization of the second pressure providing unit (1110), the buffer solution transmits the received pressure to the selective concentration filter (1400). In other words, the analyte concentrated in the selective permeable membrane (1410) of the selective concentration filter (1400) according to the additionally applied pressure is discharged to the outside through the first passage (1312). Here, it is sufficient to drop the concentrated analyte into the reservoir of the diagnostic kit. In addition, the first pressure providing unit (1120) may further comprise a first packing (1122), and the second pressure providing unit (1110) may further comprise a second packing (1112). The packing may be applied to increase the sealing force of the channel when transmitting the pressure.

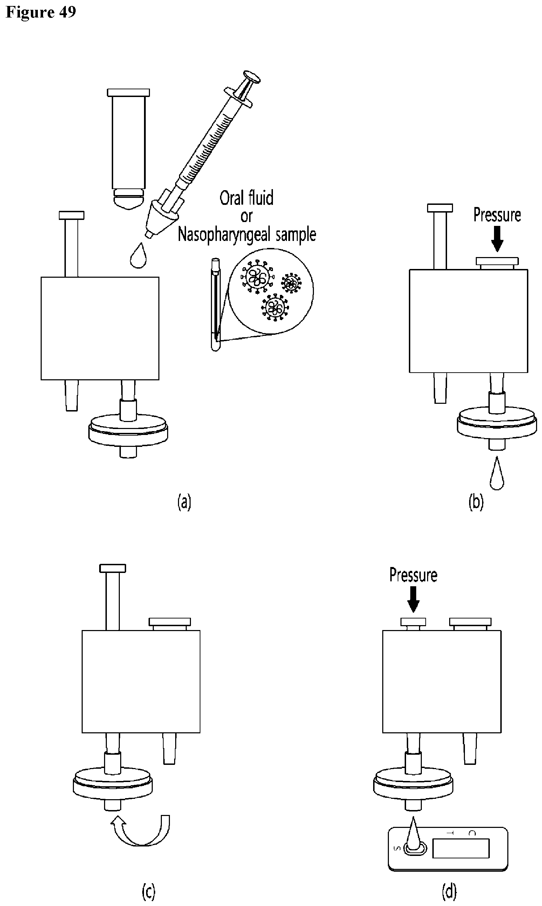

Figure 49 is a reference diagram illustrating a sample concentration and concentrated sample supply process using a sample concentration device according to one embodiment of the present invention. Step (a) is a step of coupling a selective concentration device (selective concentration unit) to the body, and introducing a sample and (optionally) a buffer solution into the first channel. Step (b) is a process of pressurizing the sample through the first pressure providing unit, thereby discharging at least a portion of the remaining substances to the outside, and concentrating the analyte on the selective permeable membrane. The pressure can be applied by hand, but can also be applied to the concentration using a negative pressure-based method such as a vacuum tube used for blood collection. In addition, concentration using a dip stick assay, etc. is also possible, but is not necessarily limited thereto.

Step (c) is a step of separating the selective concentration device from the body, reversing the direction, and then connecting the selective concentration device to another coupling unit of the body. Step (d) is a step of introducing a buffer solution, purified water, etc. into the second channel, and pressurizing through the second pressure providing unit, thereby supplying the analytes concentrated on the selective permeable membrane to the outside, particularly to the injection unit of the LFA kit.

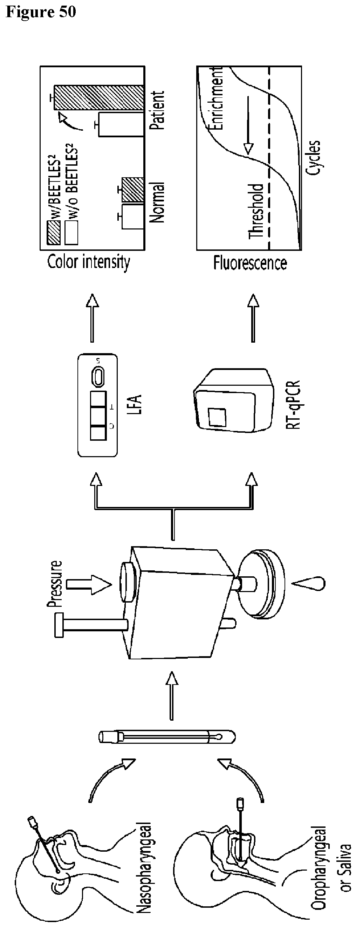

Figure 50 illustrates an example of a field of use of the selective concentration filter of the present invention and a sample concentration device comprising the same. Through an effective pressurization process of BEETLES[2], a selective concentration filter included in a sample concentration device according to one embodiment of the present invention, a sample can be concentrated, and the concentrated sample can be provided to a diagnostic kit or a molecular diagnostic device. In addition, by using the sample concentration device of the present invention, not only immunodiagnosis (LFA) but also molecular diagnosis (RT-qPCR) can be applied to nasopharyngeal, oropharyngeal, and saliva specimens through preprocessing.

According to one embodiment of the present invention described above, BEETLES[2] is an effective selective concentration filter for COVID-19 diagnosis. Since BEETLES[2], a hybrid filter composed of RBCM on an AAO membrane, has permeability, selectivity and tunability, biomolecules to be concentrated can be effectively and selectively concentrated. In addition, by combining LFA and smartphone-based AI technology with the support of BEETLES[2], clinical results can be easily determined based on AI.

BEETLES[2] according to one embodiment of the present invention has permeability and selectivity, and as described above, has permeability and tunability under a certain pressure. BEETLES[2] has permeability and tunability depending on pressure, molecular size, charge, and buffer conditions. The present filter has a dependency on pressure, and especially when using BSA (bovine serum albumin), negatively charged BSA has the advantage of being able to regulate the concentrated concentration depending on the pressure. In addition, in the case of BSA, there is a surface charge effect (permeability). In other words, at low pH, it is concentrated as positively charged BSA (pI: 4.5 to 4.8), and at higher pH, there is no concentration (neutral and negative charge conditions).

These examples are intended to explain the technical idea of the present embodiment, and the scope of the technical idea of the present embodiment is not limited by these examples. The scope of protection of the present embodiment should be interpreted by the claims below, and all technical ideas within the equivalent scope should be interpreted as being included in the scope of rights of the present embodiment.

**Claims**

1. A selective concentration filter comprising:

   a base having pores; and
   a selective permeable membrane which is coated on the base and which comprises vesicles, wherein the selective permeable membrane concentrates an analyte to be analyzed from a sample and allows at least a portion of the remaining substances, other than the analyte, to be permeated through a pore structure.

2. The selective concentration filter according to claim 1, wherein the surface of the selective permeable membrane is hydrophilic and has a negative charge.

3. The selective concentration filter according to claim 1, wherein the pore is selected so as to concentrate the analyte by considering at least one property selected from the group consisting of the size of the analyte, the charge of the analyte, and the affinity with the solvent, and the diameter of the pore is in the range of 10 nm to 150 m.

4. The selective concentration filter according to claim 1, wherein the remaining substance of the sample comprises a fixed separation substance and a dynamic separation substance, and the degree of permeation of the dynamic separation substance depends on the spacing of the phospholipids of the cell membrane component in a first pressure section or a second pressure section distinguished according to a reference pressure applied to the selective permeable membrane.

5. The selective concentration filter according to claim 4, wherein the remaining substance of the sample further comprises a buffer solution, and the degree of permeation of the dynamic separation substance depends on the hydrogen ion concentration of the buffer solution in a first hydrogen ion concentration section or a second hydrogen ion concentration section distinguished according to the reference hydrogen ion concentration.

6. A sample concentration device comprising:

   a selective concentration unit comprising a selective concentration filter and a concentration filter holder surrounding the selective concentration filter; and

a body comprising a coupling unit that is coupled to the selective concentration filter as necessary, and having a channel formed therein for supplying a sample to be concentrated to the selective concentration filter module, wherein the selective concentration filter comprises:

a base having pores; and
a selective permeable membrane which is coated on the base and which comprises vesicles, wherein the selective permeable membrane does not allow an analyte to be analyzed from a sample to be permeated and allows at least a portion of the remaining substances, other than the analyte, to be permeated through the pores.

7. The sample concentration device according to claim 6, wherein the concentration filter holder further comprises a first passage and a second passage formed in a space separated from each other for the input or discharge of the sample.

8. The sample concentration device according to claim 7, wherein the first passage is located on the selective permeable membrane of the selective concentration filter, and the second passage is located on the base.

9. The sample concentration device according to claim 6, wherein the concentration filter holder further comprises

a first holder having the first passage; and
a second holder having the second passage, and
the selective concentration filter is located between the first holder and the second holder.

10. The sample concentration device according to claim 9, wherein the sample concentration device further comprises a fixing unit that seals the selective concentration filter and a supporting unit that supports the selective concentration filter, which are located between the first holder and the second holder.

11. The sample concentration device according to claim 7, wherein the first passage is for injecting the sample, and the second passage is for discharging at least a portion of the remaining substances that has permeated through the pores to the outside, and

the sample concentration device further comprises a first pressure providing unit that applies pressure to the sample to inject the sample through the first passage and provides pressure to discharge at least a portion of the remaining substances to the outside through the second passage, and
the body further comprises a first channel through which the first pressure providing unit can move.

12. The sample concentration device according to claim 11, wherein the sample concentration device further comprises a second pressure providing unit that applies pressure to the selective concentration filter through the second passage to discharge the concentrated analyte to the outside through the first passage, and
the body further comprises a second channel through which the second pressure providing unit can move.

13. The sample concentration device according to claim 12, wherein the body further comprises a coupling unit that surrounds and supports the first pressure providing unit and the second pressure providing unit, and can be selectively coupled to the first passage or the second passage.

14. The sample concentration device according to claim 6, wherein the surface of the selective permeable membrane is hydrophilic and has a negative charge, and
the pore is selected so as to concentrate the analyte by considering at least one property selected from the group consisting of the size of the analyte, the charge of the analyte, and the affinity with the solvent.

15. The sample concentration device according to claim 6, wherein the remaining substance of the sample comprises a fixed separation substance and a dynamic separation substance, and the degree of permeation of the dynamic separation substance depends on the spacing of the phospholipids of the cell membrane component in a first pressure section or a second pressure section distinguished according to a reference pressure applied to the selective permeable membrane.

16. The sample concentration device according to claim 15, wherein the remaining substance of the sample further comprises a buffer solution, and

the degree of permeation of the dynamic separation substance depends on the hydrogen ion concentration of the buffer solution in a first hydrogen ion concentration section or a second hydrogen ion concentration section distinguished according to the reference hydrogen ion concentration.

17. A method for manufacturing a selective concentration filter, comprising:

a step of preparing a cell membrane mixture solution comprising a cell membrane comprising a vesicle and a buffer solution;
a step of applying the cell membrane mixture solution to a base having pores; and
a step of evaporating at least a portion of the buffer solution through heat treatment and fixing the cell membrane on the base.

18. The method for manufacturing a selective concentration filter according to claim 17, wherein the cell membrane vesicles are obtained by ultrasonicating red blood cell membranes, and the cell membrane vesicles in the cell membrane mixture solution are 1% (v/v) to 4% (v/v).

19. The method for manufacturing a selective concentration filter according to claim 17, wherein the method further comprises, following the coating step, a step of heat treating at 40 °C to 60 °C.

20. A method for concentrating a sample using a selective concentration device for a sample that comprises a selective concentration filter; and a concentration filter holder surrounding the selective concentration filter, wherein

the selective concentration filter comprises: a base having pores; and a selective permeable membrane which is coated on the base and which comprises vesicles, wherein the selective permeable membrane does not allow an analyte to be analyzed from a sample to be permeated and allows at least a portion of the remaining substances, other than the analyte, to be permeated through the pores, and the concentration filter holder comprises a first passage and a second passage for the input or discharge of the sample, and
the method for concentrating a sample comprises:

a step of injecting the sample through the first passage and discharging at least a portion of the remaining substances that has permeated through the pore structure to the outside through the second passage; and
a step of applying pressure to the selective concentration filter through the second passage to discharge at least a portion of the analyte concentrated in the selective permeable membrane through the first passage.

**Figure 1**

```
                    ┌───────────┐
                    │   Start   │
                    └─────┬─────┘
                          │
                          ▼
┌──────────────────────────────────────────────────────┐
│              Prepare cell membrane                     │─── S10
└──────────────────────────┬─────────────────────────────┘
                          │
                          ▼
┌──────────────────────────────────────────────────────┐
│   Dilute cell membrane with purified water so as to    │─── S20
│      provide cell membrane mixture solution            │
└──────────────────────────┬─────────────────────────────┘
                          │
                          ▼
┌──────────────────────────────────────────────────────┐
│   Apply ultrasonic waves to cell membrane mixture      │─── S30
│    solution so as to form cell membrane vesicles       │
└──────────────────────────┬─────────────────────────────┘
                          │
                          ▼
┌──────────────────────────────────────────────────────┐
│  Apply, to base having pore structure, cell membrane   │─── S40
│  mixture solution in which cell membrane vesicles are  │
│  formed                                                │
└──────────────────────────┬─────────────────────────────┘
                          │
                          ▼
┌──────────────────────────────────────────────────────┐
│  Induce cell fusion through heat treatment and apply   │─── S50
│  to base                                               │
└──────────────────────────┬─────────────────────────────┘
                          │
                          ▼
                    ┌───────────┐
                    │    End    │
                    └───────────┘
```

Figure 2

**Figure 3**

whole blood

whole blood separation

plasma

white blood cell and platelet

red blood cell

red blood cell disruption

cell membrane separation

**Figure 4**

Cell Culture

cell disruption

cell membrane separation

**Figure 5**

(a) 0.1%

(b) 0.5%

(c) 1%

(d) 2%

(e) 4%

**Figure 6**

(a) 0.1%

(b) 0.25%

(c) 0.5%

(d) 1%

(e) 2%

**Figure 7**

(a) 0.1%

(d) 2%

(b) 0.1%

(e) 2%

(c) 0.1%

(f) 2%

Figure 8

Figure 9

Figure 10

Figure 11

Height

Surface potential

Figure 12

**Figure 13**

**Figure 14**

**Figure 15**

sample concentration device (10)

control unit (600)

pressure providing unit (400)

pH adjusting unit (500)

sample supply unit (300)

selective concentration filter (100)

concentration filter holder (200)

**Figure 16**

**Figure 17**

220

210

215

**Figure 18**

**Figure 19**

concentrated sample

permeated buffer outlet

**Figure 20**

selective concentration filter (100)

selective permeable membrane (120)

base (110)

**Figure 21**

cell membrane

selective permeable membrane

base

**Figure 22**

25 mm

Bare AAO membrane
(before cell membrane coating)

2% EM-coated AAO membrane
(after cell membrane coating)

**Figure 23**

(a)

(b)

(c)

Figure 24

(a) COVID-19 Ag (ng/mL)

(b) COVID-19 Ab (AU/mL)

(c) COVID-19 Ag (ng/mL)

**Figure 25**

(a) 0.1 ng/mL Ref.

(b) 0.1 ng/mL Prec.

(c) 1 ng/mL Ref.

**Figure 26**

**Figure 27**

**Figure 28**

**Figure 29**

(a)

(b)

(c)

(d)

Figure 30

EP 4 534 186 A1

|  | size property MW(size) | charge property (charge) | hydrophobicity/ hydrophilicity property (hydrophobicity) |
|---|---|---|---|
| COVID-19 NP | 45kDa | positive | Hydrophobic |
| Influenza(H2N3) NP | 56 kDa | positive | Hydrophobic |
| IgG | 150 kDa | negative | Hydrophobic |
| polystyrene bead | 20 nm | negative | Hydrophobic |
| Fluorescence sodium salt | 0.412kDa | positive |  |
| Cytochrome c | 12.4 kDa | positive | Hydrophilic |
| BSA | 66.5 kDa(7.1 nm) | negative | Hydrophilic |
| Glucose oxidase | 80 kDa (6*5*7.7 nm) | negative | Hydrophobic |
| hCG | 36.7 kDa | negative | Hydrophilic |

concentrated { COVID-19 NP, Influenza(H2N3) NP, IgG, polystyrene bead }

unconcentrated { Fluorescence sodium salt, Cytochrome c, BSA, Glucose oxidase, hCG }

(3bar)

**Figure 31**

IgG (MW ~ 150 kDa)

**Figure 32**

# Influenza A

| Sample | Permeate | Preconcentrated |

**Figure 33**

|  | first pressure section (non-permeated) | second pressure section (permeated) |
|---|---|---|
| target 1 | $X_1 \sim X_2$ | $X_3 \sim X_4$ |
| ⋮ | ⋮ | ⋮ |
| target N | $X_{N1} \sim X_{N2}$ | $X_{N3} \sim X_{N4}$ |

Figure 34

Figure 35

**Figure 36**

**Figure 37**

$$\Delta P \approx 0$$

(a)

$$\Delta P > 0$$

(b)

**Figure 38**

(a) 0.1 bar

(b) 1 bar

Figure 39

| | first pH section (non-permeated) | second pH section (permeated) |
|---|---|---|
| target 1 | $Y_1 \sim Y_2$ | $Y_3 \sim Y_4$ |
| $\vdots$ | $\vdots$ | $\vdots$ |
| target 2 | $Y_{N1} \sim Y_{N2}$ | $Y_{N3} \sim Y_{N4}$ |

Figure 40

**Figure 41**

BSA ( pI ~ 4.9 )

**Figure 42**

(a)  (b)  (c)

Figure 43

EP 4 534 186 A1

**Figure 44**

**Figure 45**

**Figure 46**

**Figure 47**

**Figure 48**

**Figure 49**

(a)

(b)

(c)

(d)

**Figure 50**

## INTERNATIONAL SEARCH REPORT

| International application No. |
|---|
| **PCT/KR2022/014223** |

**A.      CLASSIFICATION OF SUBJECT MATTER**

**B01D 67/00**(2006.01)i; **B01D 71/06**(2006.01)i; **C07K 1/34**(2006.01)i; **C07K 14/765**(2006.01)i

According to International Patent Classification (IPC) or to both national classification and IPC

**B.      FIELDS SEARCHED**

Minimum documentation searched (classification system followed by classification symbols)

B01D 67/00(2006.01); B01D 5/00(2006.01); B01D 53/22(2006.01); B01D 61/48(2006.01); B01D 61/54(2006.01); B01J 20/20(2006.01); B81B 1/00(2006.01); G01N 1/40(2006.01); G01N 33/68(2006.01); G01N 37/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 포어(pore), 베이스(base), 소낭(vesicle), 선택적 투과막(selective permeable membrane), 농축(enrichment)

**C.      DOCUMENTS CONSIDERED TO BE RELEVANT**

| Category* | Citation of document, with indication, where appropriate, of the relevant passages | Relevant to claim No. |
|---|---|---|
| X | KR 10-1577524 B1 (KWANGWOON UNIVERSITY INDUSTRY-ACADEMIC COLLABORATION FOUNDATION et al.) 15 December 2015 (2015-12-15)<br>See paragraphs [0031]-[0041]; claims 1 and 11; and figures 1-2. | 1-3 |
| Y | | 6,14 |
| A | | 4-5,7-13,15-20 |
| Y | KR 10-2015-0143002 A (KOREA UNIVERSITY RESEARCH AND BUSINESS FOUNDATION) 23 December 2015 (2015-12-23)<br>See claims 3-4. | 6,14 |
| A | KR 10-2009-0014366 A (GENERAL ELECTRIC COMPANY) 10 February 2009 (2009-02-10)<br>See paragraphs [0027]-[0036]; and figures 1a-1d. | 1-20 |
| A | KR 10-1734943 B1 (KOREA SOUTH-EAST POWER CO., LTD.) 12 May 2017 (2017-05-12)<br>See paragraphs [0078]-[0097]; and figures 6-7. | 1-20 |

[✓] Further documents are listed in the continuation of Box C.      [✓] See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **23 February 2023** | **24 February 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.

**PCT/KR2022/014223**

**C. DOCUMENTS CONSIDERED TO BE RELEVANT**

| Category* | Citation of document, with indication, where appropriate, of the relevant passages | Relevant to claim No. |
|---|---|---|
| A | KR 10-2008-0113014 A (TOSHIBA MITSUBISHI-ELECTRIC INDUSTRIAL SYSTEMS CORPORATION) 26 December 2008 (2008-12-26) See claims 1-17. | 1-20 |

Form PCT/ISA/210 (second sheet) (July 2022)

**INTERNATIONAL SEARCH REPORT**
Information on patent family members

International application No.

**PCT/KR2022/014223**

| Patent document cited in search report | | | Publication date (day/month/year) | Patent family member(s) | | | Publication date (day/month/year) |
|---|---|---|---|---|---|---|---|
| KR | 10-1577524 | B1 | 15 December 2015 | None | | | |
| KR | 10-2015-0143002 | A | 23 December 2015 | None | | | |
| KR | 10-2009-0014366 | A | 10 February 2009 | AU | 2007-257074 | A1 | 13 December 2007 |
| | | | | AU | 2007-257074 | B2 | 21 June 2012 |
| | | | | BR | PI0711522 | A2 | 01 November 2011 |
| | | | | CA | 2653014 | A1 | 13 December 2007 |
| | | | | CA | 2653014 | C | 24 November 2015 |
| | | | | CN | 101454067 | A | 10 June 2009 |
| | | | | CN | 101454067 | B | 04 July 2012 |
| | | | | EP | 2029262 | A2 | 04 March 2009 |
| | | | | EP | 2029262 | B1 | 13 June 2012 |
| | | | | ES | 2387414 | T3 | 21 September 2012 |
| | | | | JP | 2009-539578 | A | 19 November 2009 |
| | | | | JP | 4913212 | B2 | 11 April 2012 |
| | | | | MX | 2008015223 | A | 12 December 2008 |
| | | | | MY | 146075 | A | 29 June 2012 |
| | | | | TW | 200803967 | A | 16 January 2008 |
| | | | | TW | I432254 | B | 01 April 2014 |
| | | | | US | 2007-0278099 | A1 | 06 December 2007 |
| | | | | US | 7427342 | B2 | 23 September 2008 |
| | | | | WO | 2007-143296 | A2 | 13 December 2007 |
| | | | | WO | 2007-143296 | A3 | 28 February 2008 |
| KR | 10-1734943 | B1 | 12 May 2017 | None | | | |
| KR | 10-2008-0113014 | A | 26 December 2008 | CN | 101389387 | A | 18 March 2009 |
| | | | | CN | 101389387 | B | 28 August 2013 |
| | | | | EP | 2036600 | A1 | 18 March 2009 |
| | | | | EP | 2036600 | B1 | 27 February 2019 |
| | | | | JP | 2014-132212 | A | 17 July 2014 |
| | | | | JP | 5456312 | B2 | 26 March 2014 |
| | | | | JP | 5665953 | B2 | 04 February 2015 |
| | | | | TW | 200829324 | A | 16 July 2008 |
| | | | | TW | I329031 | A | 21 August 2010 |
| | | | | TW | I329031 | B | 21 August 2010 |
| | | | | US | 2010-0162752 | A1 | 01 July 2010 |
| | | | | US | 2013-0291718 | A1 | 07 November 2013 |
| | | | | US | 8500874 | B2 | 06 August 2013 |
| | | | | US | 8608832 | B2 | 17 December 2013 |
| | | | | WO | 2008-004278 | A1 | 10 January 2008 |
| | | | | WO | 2008-004321 | A1 | 03 December 2009 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020210124410
- KR 1020210129131
- KR 1020190127690